# EUROPEAN PATENT APPLICATION

(11) **EP 4 299 751 A1**
(43) Date of publication of application: **03.01.2024**
(21) Application number: 22767122.9
(22) Date of filing: 08.03.2022
(51) Int. Cl.: C12N 15/864, C12N 7/01, C12N 7/02

(54) **METHOD FOR PRODUCING RECOMBINANT AAV9 VIRION**

(30) Priority: 09.03.2021 JP 2021037870
(71) Applicant: JCR Pharmaceuticals Co., Ltd., Ashiya-shi, Hyogo 659-0021 (JP)
(72) Inventor: OHTSUKI, Kota, Kobe-shi, Hyogo 651-2241 (JP); HIRASHIMA, Tomoki, Kobe-shi, Hyogo 651-2241 (JP)
(74) Representative: Boult Wade Tennant LLP
(86) International application number: PCT/JP2022/009902
(87) International publication number: WO 2022/191168

(57) **Abstract**

A production method of recombinant adeno-associated virus particles (rAAV virions) having less empty viral contamination is disclosed. The production method includes: (a) culturing mammalian cells containing an introduced AAV vector in a medium to release rAAV virions into the medium; (b) recovering a culture supernatant from the medium; and (c) purifying the rAAV virions from the recovered culture supernatant by affinity column chromatography using a material having an affinity for an AAV capsid protein as a stationary phase and anion column chromatography.

## Description

### Technical Field

The present invention relates to a production method of recombinant adeno-associated virus particles (rAAV virions) that can be used for gene therapy, and more particularly, to a production method of rAAV9 virions, the method including a step of purifying rAAV9 virions by affinity column chromatography and anion column chromatography.

### Background Art

Adeno-associated viruses (AAV) belong to the Parvoviridae family, the smallest class of linear single-stranded DNA viruses in nature, and a viral genome thereof is about 4.7 kb. It does not have an envelope and forms regular icosahedron particles having a diameter of about 22 nm.

In AAV, there are many mutants having different serotypes called serotypes, and it is known that 11 types of serotypes among them infect human cells. Each of the serotypes of AAV has directivity specific to an organ. For example, it is known that AAV9 infects central nervous system (CNS), heart, lung, liver, and skeletal muscle with directivity.

Although AAV infects various human organs, it is considered that AAV is not pathogenic. Therefore, attempts have been made to utilize AAV as a material for producing a recombinant virus for gene therapy. Since each serotype of AAV has directivity in the organ to be infected, it has also been attempted to selectively use the serotype depending on an organ into which a gene is to be introduced by gene therapy. Since AAV9 has directivity in the central nervous system (CNS), heart, lung, liver, and skeletal muscle, it has also been attempted to use AAV9 as a material for producing a recombinant virus for introducing a gene into these organs and tissues.

In gene therapy to which AAV is applied, a recombinant AAV genome (rAAV genome) in which a part of a wild-type AAV genome is substituted with a foreign gene is administered to a patient in the form of a recombinant AAV virion (rAAV virion) encapsulated in a capsid protein. The rAAV virion is produced by performing a process in which a wild-type AAV genome is encapsulated in a capsid protein to form a wild-type AAV in a test tube. In this process, the rAAV genome is encapsulated in the capsid protein to form an rAAV virion, but an empty particle (empty capsid) encapsulating no rAAV virion is also formed. In particular, it has been reported that nearly 80% of empty capsids are contained in transient expression systems of plasmid DNA. (Non Patent Literature 1). The empty capsids are impurities that occur in the process of producing rAAV virions used for gene therapy.

As a method for removing the empty capsids, a method of separating rAAV virions encapsulating DNA and empty capsids encapsulating no DNA by density gradient ultracentrifugation on a small scale is known (Non Patent Literature 2). However, the rAAV virions used in the clinical trial of gene therapy using an AAV vector do not necessarily have the empty capsids removed, and rAAV virions (scAAV2/8-FIX) actually administered in the clinical trial performed on hemophilia B patients have a ratio of the empty capsids and the rAAV virions of about 4:1, and contain only about 20% of the rAAV virions (Non Patent Literatures 1 and 3).

Examples of a scale-up empty capsid removal method include column chromatography using an anion exchanger (Patent Literature 1). In addition, a method for separating rAAV5 (rAAV virions of serotype 5) from empty capsids by two-step ion exchange column chromatography has been reported (Non Patent Literature 4). However, the method cannot be applied to rAAV2 (rAAV virions of serotype 2).

### Citation List

### Patent Literature

Patent Literature 1: Japanese Patent No. 5956331

### Non Patent Literature

Non Patent Literature 1: James A. Human Gene Therapy. 22. 595-604 (2011)
Non Patent Literature 2: Ayuso E. Gene Therapy. 17. 503-510 (2010)
Non Patent Literature 3: Maria S. Human Gene Therapy Methods. 28. 101-108 (2017)
Non Patent Literature 4: Nicole B. Molecular Therapy. 6. 678-686 (2002)

### Summary of Invention

### Technical Problem

An object of the present invention is to provide a method for efficiently purifying rAAV9 virions that can be used for introducing a gene encoding a desired protein into a cell in gene therapy or the like.

### Solution to Problem

In the studies aimed at the above object, as a result of intensive studies, the present inventors have found that an empty capsid and an rAAV9 virion can be efficiently separated from an rAAV9 virion solution containing an empty capsid by using anion exchange column chromatography, thereby completing the present invention. That is, the present invention includes the following.
1. A production method of rAAV9 virions, the production method including:
   (a) culturing mammalian cells containing an introduced AAV vector in a medium to release rAAV9 virions into the medium;
   (b) recovering a culture supernatant from the medium; and
   (c) purifying the rAAV9 virions from the recovered culture supernatant by affinity column chromatography using a material having an affinity for an AAV capsid protein as a stationary phase and anion column chromatography using an anion exchanger as a stationary phase.
2. The production method according to 1, in which the stationary phase used for the affinity column chromatography has a specific affinity for an AAV capsid protein of serotype 9.
3. The production method according to 1 or 2, in which the anion exchanger is a strong anion exchanger.
4. The production method according to 3, in which the strong anion exchanger contains a quaternary amine.
5. The production method according to any one of 1 to 4, in which in the anion exchange column chromatography, the rAAV9 virions are adsorbed on the column, and then the rAAV9 virions are eluted from the column by a buffer solution containing a quaternary ammonium salt to recover a fraction containing the rAAV9 virions.
6. The production method according to 5, in which a pH of the buffer solution is 8.5 to 10.5.
7. The production method according to 5 or 6, in which a buffer contained in the buffer solution is selected from the group consisting of tris-hydrochloric acid, Bis-Tris, Bis-Tris propane, bicine (N,N-bis(2-hydroxyethyl)glycine), 4-2-hydroxyethyl-1-piperazineethanesulfonic acid (HEPES), 3-{ [tris(hydroxymethyl)methyl] amino } propane ethanesulfonic acid (TAPS), tricine (N-tris(hydroxymethyl)methylglycine), and a combination of any two or more thereof.
8. The production method according to 5 or 6, in which a buffer contained in the buffer solution is Bis-Tris propane.
9. The production method according to 7 or 8, in which a concentration of the buffer is 10 mM to 30 mM.
10. The production method according to any one of 5 to 9, in which the buffer solution contains a neutral salt.
11. The method according to 10, in which the neutral salt is magnesium chloride.
12. The method according to 10 or 11, in which a concentration of the neutral salt is 1 mM to 10 mM.
13. The production method according to any one of 5 to 12, in which the buffer solution contains a nonionic surfactant.
14. The production method according to 13, in which the nonionic surfactant is selected from the group consisting of a polyoxyethylene-polyoxypropylene block copolymer, a polysorbate, a poloxamer, and a combination of any two or more thereof.
15. The production method according to 13, in which the nonionic surfactant is selected from the group consisting of polysorbate 20, polysorbate 80, poloxamer 188, and a combination of any two or more thereof.
16. The production method according to any one of 13 to 15, in which a concentration of the nonionic surfactant is 0.0002 to 0.01% (w/v).
17. The production method according to any one of 5 to 16, in which the quaternary ammonium salt contained in the buffer solution is a tetramethylammonium salt.
18. The production method according to 17, in which the tetramethylammonium salt is tetramethylammonium chloride.
19. The production method according to any one of 5 to 18, in which the adsorbed rAAV9 virions are eluted by the buffer solution containing the quaternary ammonium salt at a concentration of 20 mM to 60 mM.
20. The production method according to any one of 5 to 18, in which the adsorbed rAAV9 virions are eluted by the buffer solution containing the quaternary ammonium salt at a concentration of 35 mM to 55 mM.
21. The production method according to any one of 5 to 20, in which in the anion exchange column chromatography, a fraction containing the rAAV9 virions is recovered so that a ratio of the rAAV9 virions and empty capsids is 2 : 8 to 10 : 0.
22. The production method according to any one of 5 to 20, in which in the anion exchange column chromatography, a fraction containing the rAAV9 virions is recovered so that a ratio of the rAAV9 virions and empty capsids is 5 : 5 to 10 : 0.
23. The production method according to any one of 5 to 20, in which in the anion exchange column chromatography, a fraction containing the rAAV9 virions is recovered so that a ratio of the rAAV9 virions and empty capsids is 7 : 3 to 10 : 0.
24. A production method of rAAV9 virions, the production method including loading an aqueous solution containing rAAV9 virions and empty capsids onto anion exchange column chromatography using an anion exchanger as a stationary phase to adsorb the rAAV9 virions on the anion exchanger, and then eluting the rAAV9 virions from the anion exchanger by a buffer solution containing a quaternary ammonium salt to recover a fraction containing the rAAV9 virions.
25. The production method according to 24, in which a pH of the buffer solution is 8.5 to 10.5.
26. The production method according to 24 or 25, in which a buffer contained in the buffer solution is selected from the group consisting of tris-hydrochloric acid, Bis-Tris, Bis-Tris propane, bicine (N,N-bis(2-hydroxyethyl)glycine), 4-2-hydroxyethyl-1-piperazineethanesulfonic acid (HEPES), 3-{[tris(hydroxymethyl)methyl]amino}propane ethanesulfonic acid (TAPS), tricine (N-tris(hydroxymethyl)methylglycine), and a combination of any two or more thereof.
27. The production method according to 24 or 25, in which a buffer contained in the buffer solution is Bis-Tris propane.
28. The production method according to 26 or 27, in which a concentration of the buffer is 10 mM to 30 mM.
29. The production method according to any one of 24 to 28, in which the buffer solution contains a neutral salt.
30. The method according to 29, in which the neutral salt is magnesium chloride.
31. The method according to 29 or 30, in which a concentration of the neutral salt is 1 mM to 10 mM.
32. The production method according to any one of 24 to 31, in which the buffer solution contains a nonionic surfactant.
33. The production method according to 32, in which the nonionic surfactant is selected from the group consisting of a polyoxyethylene-polyoxypropylene block copolymer, a polysorbate, a poloxamer, and a combination of any two or more thereof.
34. The production method according to 32, in which the nonionic surfactant is selected from the group consisting of polysorbate 20, polysorbate 80, poloxamer 188, and a combination of any two or more thereof.
35. The production method according to any one of 32 to 34, in which a concentration of the nonionic surfactant is 0.0002 to 0.01% (w/v).
36. The production method according to 24 to 35, in which the quaternary ammonium salt contained in the buffer solution is a tetramethylammonium salt.
37. The method according to 36, in which the tetramethylammonium salt is tetramethylammonium chloride.
38. The production method according to any one of 24 to 37, in which the adsorbed rAAV9 virions are eluted by the buffer solution containing the quaternary ammonium salt at a concentration of 20 mM to 60 mM.
39. The production method according to any one of 24 to 37, in which the adsorbed rAAV9 virions are eluted by the buffer solution containing the quaternary ammonium salt at a concentration of 35 mM to 55 mM.
40. The production method according to any one of 24 to 39, in which a fraction containing the rAAV9 virions is recovered so that a ratio of the rAAV9 virions and empty capsids is 2 : 8 to 10 : 0.
41. The production method according to any one of 24 to 39, in which a fraction containing the rAAV9 virions is recovered so that a ratio of the rAAV9 virions and empty capsids is 5 : 5 to 10 : 0.
42. The production method according to any one of 24 to 39, in which a fraction containing the rAAV9 virions is recovered so that a ratio of the rAAV9 virions and empty capsids is 7 : 3 to 10 : 0.

### Advantageous Effects of Invention

According to the present invention, for example, a large amount of rAAV9 virions can be inexpensively supplied to the market.

### Brief Description of Drawings

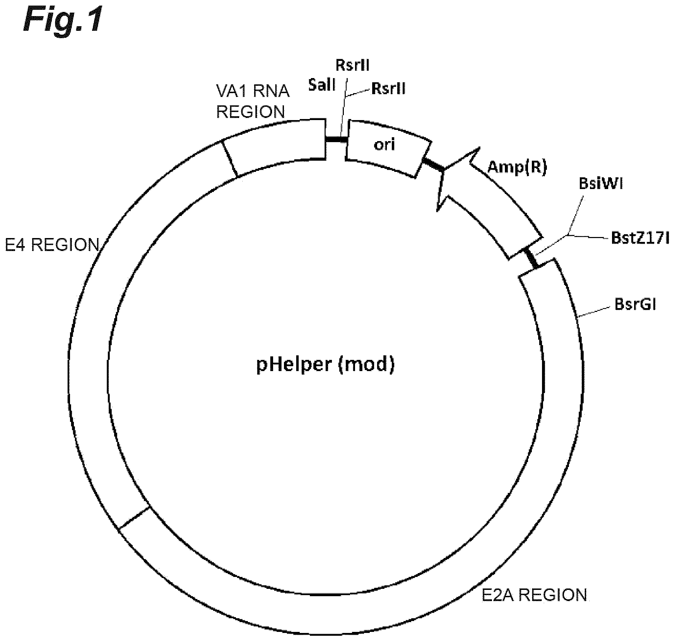

FIG. 1 is a schematic diagram showing a structure of a pHelper(mod) vector.

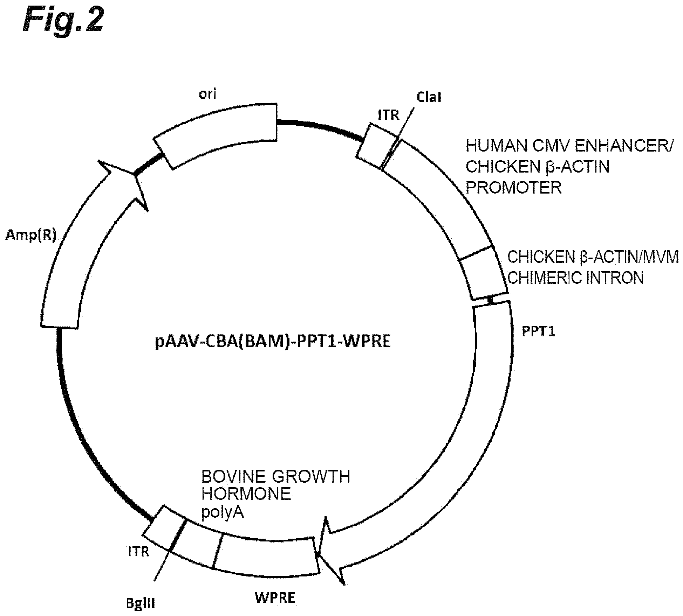

FIG. 2 is a schematic diagram showing a structure of a pAAV-CBA(BAM)-PPT1-WPRE vector.

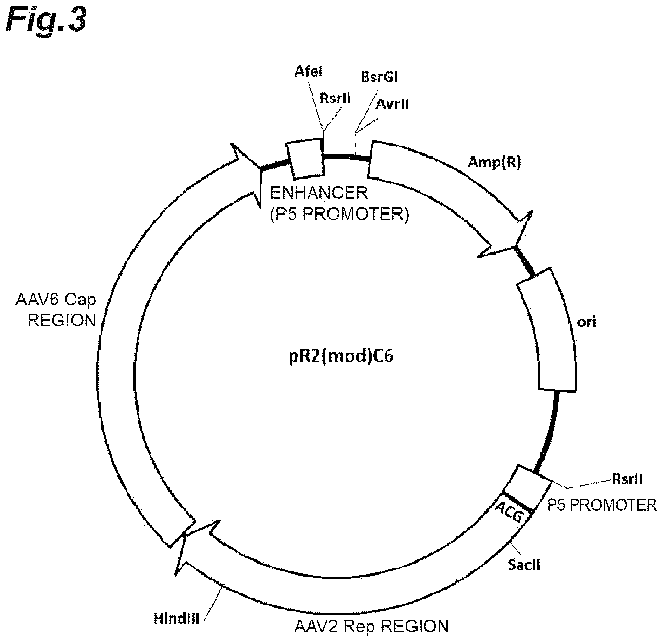

FIG. 3 is a schematic diagram showing a structure of a pR2(mod)C6 vector.

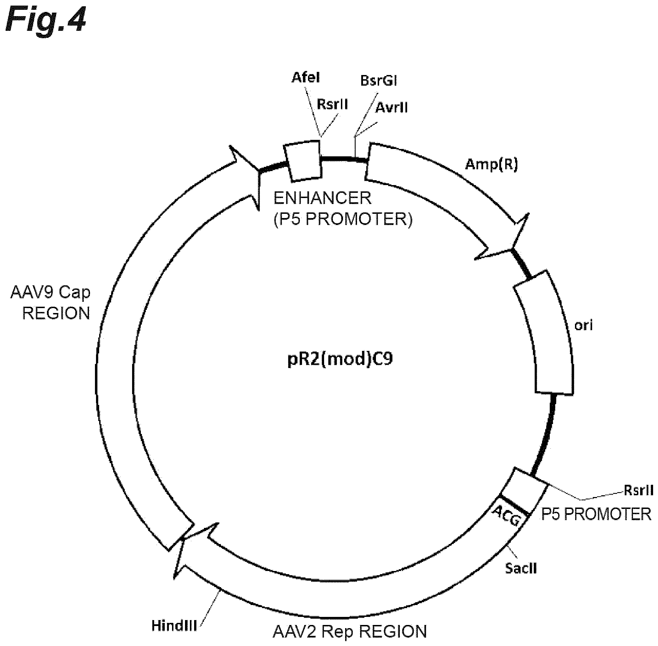

FIG. 4 is a schematic diagram showing a structure of a pR2(mod)C9 vector.

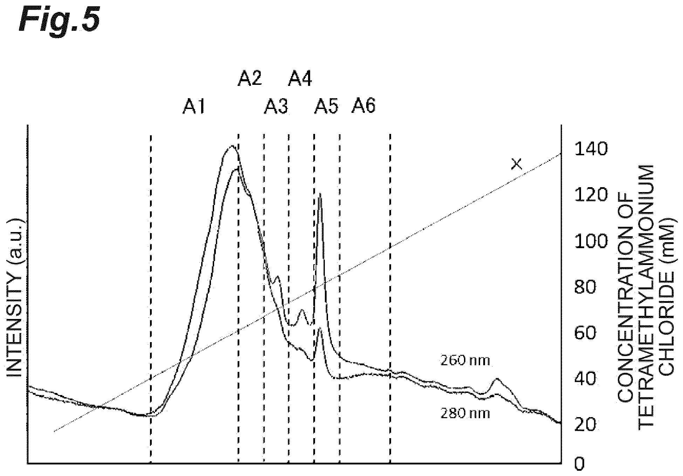

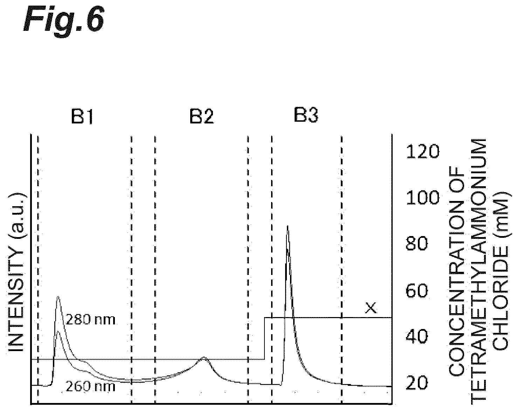

FIG. 5 is a partially enlarged view of a chromatogram obtained in a purification process (condition A) by anion exchange column chromatography described in Example 8 (detection wavelengths: 260 nm and 280 nm), in which A1 to A6 indicate eluted fractions, respectively, and the horizontal axis indicates a time axis, the left vertical axis indicates a light absorption intensity (a.u.: arbitrary unit), the right vertical axis indicates a concentration (mM) of tetraethylammonium chloride, and a change in concentration of tetraethylammonium chloride is indicated by the solid line X (hereinafter, the same applies to FIGS. 6 to 10).
FIG. 6 is a partially enlarged view of a chromatogram obtained in a purification process (condition B) by anion exchange column chromatography described in Example 9 (detection wavelengths: 260 nm and 280 nm), in which fractions containing eluates corresponding to the peaks represented by B1 to B3 are recovered as eluted fractions B1 to B3, respectively.

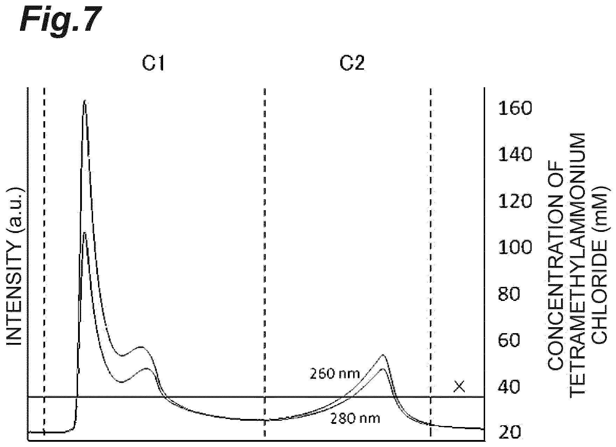

FIG. 7 is a partially enlarged view of a chromatogram obtained in a purification process (condition C) by anion exchange column chromatography described in Example 10 (detection wavelengths: 260 nm and 280 nm), in which fractions containing eluates corresponding to the peaks represented by C1 and C2 are recovered as eluted fractions C1 and C2, respectively.

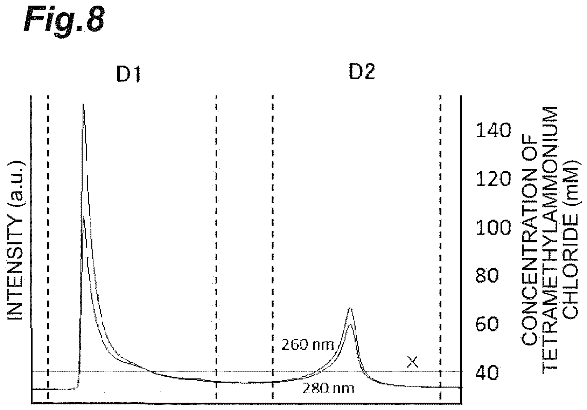

FIG. 8 is a partially enlarged view of a chromatogram obtained in a purification process (condition D) by anion exchange column chromatography described in Example 11 (detection wavelengths: 260 nm and 280 nm), in which fractions containing eluates corresponding to the peaks represented by D1 and D2 are recovered as eluted fractions D1 and D2, respectively.

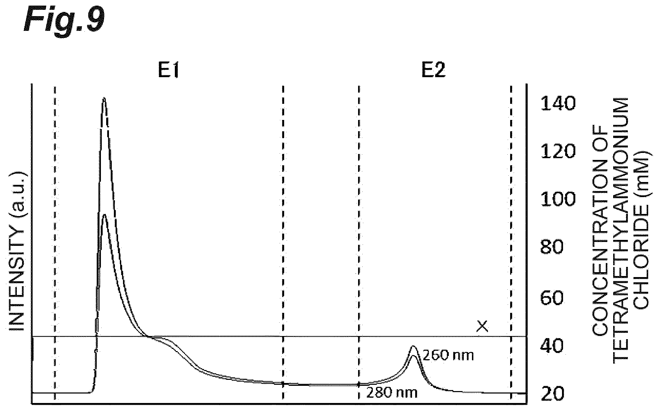

FIG. 9 is a partially enlarged view of a chromatogram obtained in a purification process (condition E) by anion exchange column chromatography described in Example 12 (detection wavelengths: 260 nm and 280 nm), in which fractions containing eluates corresponding to the peaks represented by E1 and E2 are recovered as eluted fractions E1 and E2, respectively.

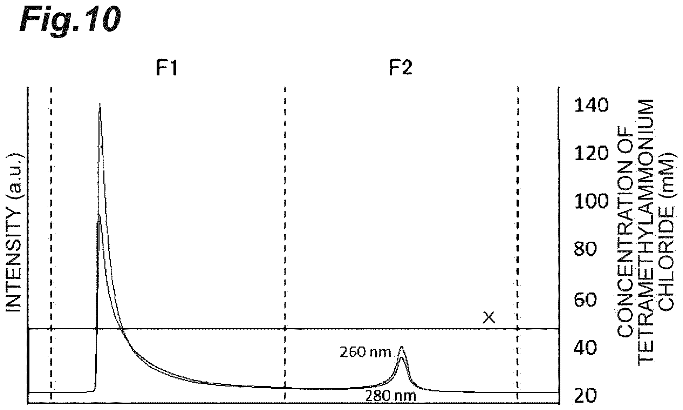

FIG. 10 is a partially enlarged view of a chromatogram obtained in a purification process (condition F) by anion exchange column chromatography described in Example 13 (detection wavelengths: 260 nm and 280 nm), in which fractions containing eluates corresponding to the peaks represented by F1 and F2 are recovered as eluted fractions F1 and F2, respectively.

### Description of Embodiments

Adeno-associated virus (AAV) has a non-enveloped regular icosahedron structure, and a capsid thereof contains three kinds of capsid proteins, VP1, VP2, and VP3, at a ratio of about 1 : 1 : 10. In wild-type AAV, on a viral genome packaged in the capsid, there are rep and cap genes sandwiched by inverted terminal repeats (ITRs) at both ends. Rep proteins (rep 78, rep 68, rep 52, and rep 40) produced from the rep gene are essential for capsid formation, and are also required for incorporation of a viral genome into a chromosome. The cap gene encodes three capsid proteins (VP1, VP2, and VP3).

In one embodiment of the present invention, the rAAV genome refers to a DNA fragment in which a part of the wild-type AAV genome is substituted with a foreign gene, and particularly refers to a DNA fragment in which the rep gene (rep region) and the cap gene (cap region) of the wild-type AAV genome are substituted with a foreign gene. The rAAV genome contains an inverted terminal repeat (ITR) upstream of the rep region and an inverted terminal repeat (ITR) downstream of the cap region of the wild-type AAV genome, and has a structure containing a foreign gene between the two ITRs. Here, a DNA strand constituting the rAAV genome may be linear or circular, or may be a single-stranded DNA or double-stranded DNA, and is preferably a linear single-stranded DNA. rAAV virions are particles in which the rAAV genome is packaged in an AAV capsid protein. A particle composed of an AAV capsid protein containing no rAAV genome is referred to as an empty capsid.

In the production of rAAV virions, three kinds of plasmids are usually used: (1) a plasmid having a structure containing a nucleotide sequence containing a first inverted terminal repeat (ITR) and a nucleotide sequence containing a second inverted terminal repeat (ITR) derived from a virus such as AAV, and a gene encoding a desired protein located between the two ITRs (plasmid 1); (2) a plasmid containing an AAV Rep gene having a function required for incorporating the nucleotide sequence of a region (including an ITR sequence) sandwiched by the ITR sequences into a genome of a host cell and a gene encoding an AAV capsid protein (plasmid 2); and (3) a plasmid containing an E2A region, an E4 region, and a VA1 RNA region of adenovirus (plasmid 3). However, the present invention is not limited thereto, and one plasmid obtained by linking two plasmids among the plasmids 1 to 3 can be used, and two kinds of plasmids of the remaining one plasmid can also be used. Furthermore, one plasmid obtained by linking the plasmids 1 to 3 can also be used.

In general, in order to produce rAAV virions, first, these three kinds of plasmids are introduced into mammalian cells to be host cells such as HEK293 cells in which an E1a gene and an E1b gene of adenovirus are incorporated into the genome by a general transfection method. Then, a region containing a nucleotide sequence containing the first inverted terminal repeat (ITR), a nucleotide sequence containing the second inverted terminal repeat (ITR), and a gene encoding a desired protein located between the two ITRs is replicated in the host cell, and the resulting single-stranded DNA is packaged in the AAV capsid protein to form an rAAV virion. Since the rAAV virion has infectivity, it is possible to introduce foreign genes into cells, tissues, and the like by administering the rAAV virions into a living body.

In one embodiment of the present invention, the AAV vector refers to a vector that contains a nucleotide sequence containing a first inverted terminal repeat (ITR) and a nucleotide sequence containing a second inverted terminal repeat (ITR) derived from the AAV genome, and can incorporate a foreign gene, which is used to produce rAAV virions. The site where a foreign gene can be incorporated into the AAV vector can be located between the first and second inverted terminal repeats (ITRs). An AAV vector into which a foreign gene is incorporated is also an AAV vector. The plasmid 1 described above corresponds to an AAV vector. The plasmids 2 and 3 themselves are not AAV vectors, and vectors in which the plasmid 1 and the plasmid 2 are linked and vectors in which the plasmid 1 and the plasmid 3 are linked are also AAV vectors. Furthermore, the plasmid 1, the plasmid 2, and the plasmid 3 are linked to form one plasmid, which is also an AAV vector. The pAAV-CBA(BAM)-PPT1-WPRE vector described in the present specification is an example of an AAV vector, and contains, between the two ITRs, a human CMV enhancer/chicken β-actin promoter, and a gene encoding human PPT1 downstream thereof.

The rAAV genome and the rAAV virion are particularly referred to as an rAAV9 genome and an rAAV9 virion, respectively, when the serotype of AAV is 9. The same applies to AAV of other serotypes.

In one embodiment of the present invention, the term "inverted terminal repeat (ITR)" refers to a nucleotide sequence which is present at the terminal of the viral genome and in which the same sequence is present repeatedly. The ITR of adeno-associated virus is a region having a chain length of about 145 nucleotides, and functions as a replication origin or the like. In one embodiment of the present invention, there are two inverted terminal repeats (ITRs) in the rAAV genome, which are referred to as a first inverted terminal repeat (ITR) and a second inverted terminal repeat (ITR), respectively. Here, when a gene encoding a foreign gene is located between two ITRs, the ITR located at the 5' side thereof is referred to as a first inverted terminal repeat (ITR), and the ITR located at the 3' side thereof is referred to as a second inverted terminal repeat (ITR). In the present invention, the inverted terminal repeat (ITR) may be derived from AAV of any serotype as long as it has at least one of a function as a replication origin and an original ITR function such as gene insertion into a host cell, but is preferably derived from AAV9.

In addition, the ITR is not limited to the wild-type ITR, and may be one obtained by adding a modification such as substitution, deletion, or addition to the nucleotide sequence of the wild-type ITR. In a case where the nucleotide of the nucleotide sequence of the wild-type ITR is substituted with another nucleotide, the number of nucleotides to be substituted is preferably 1 to 20, more preferably 1 to 10, and still more preferably 1 to 3. In a case where the nucleotide sequence of the wild-type ITR is deleted, the number of nucleotides to be deleted is preferably 1 to 20, more preferably 1 to 10, and still more preferably 1 to 3. In addition, a mutation combining substitution and deletion of these nucleotides can also be added. In a case where a nucleotide is added to the wild-type ITR, preferably 1 to 20 nucleotides, more preferably 1 to 10 nucleotides, and still more preferably 1 to 3 nucleotides are added in the ITR nucleotide sequence or to the 5' terminal or the 3' terminal. A mutation combining addition, substitution, and deletion of these nucleotides can also be added. The nucleotide sequence of the ITR to which the mutation is added shows preferably 80% or more identity, more preferably 85% or more identity, still more preferably 90% or more identity, still more preferably 95% or more identity, and further still more preferably 98% or more identity with the nucleotide sequence of the wild-type ITR.

A functional equivalent of the ITR refers to one that can be functionally used instead of the ITR. In addition, an ITR artificially constructed based on a wild-type or mutant ITR is also a functional equivalent of the ITR as long as the ITR can be substituted.

A functional equivalent of the ITR of AAV refers to one that can be functionally used instead of the ITR of AAV. In addition, an ITR artificially constructed based on a wild-type or mutant ITR of AAV is also a functional equivalent of the ITR of AAV as long as the ITR of AAV can be substituted.

In one embodiment of the present invention, the AAV vector used for producing rAAV virions contains a nucleotide sequence (1) or (2) shown below:
(1) a nucleotide sequence containing a first inverted terminal repeat (ITR) or a functional equivalent thereof, a nucleotide sequence encoding a foreign gene downstream thereof, and a nucleotide sequence containing a second inverted terminal repeat (ITR) or a functional equivalent thereof further downstream; or
(2) a nucleotide sequence containing a first inverted terminal repeat (ITR) or a functional equivalent thereof, a nucleotide sequence containing a gene expression control site downstream thereof, a nucleotide sequence encoding a foreign gene further downstream, and a nucleotide sequence containing a second inverted terminal repeat (ITR) or a functional equivalent thereof further downstream.

In one embodiment of the present invention, the rAAV genome encapsulated in the produced rAAV virion contains a nucleotide sequence (1) or (2) shown below:
(1) a nucleotide sequence containing a first inverted terminal repeat (ITR) or a functional equivalent thereof, a nucleotide sequence encoding a foreign gene downstream thereof, and a nucleotide sequence containing a second inverted terminal repeat (ITR) or a functional equivalent thereof further downstream; or
(2) a nucleotide sequence containing a first inverted terminal repeat (ITR) or a functional equivalent thereof, a nucleotide sequence containing a gene expression control site downstream thereof, a nucleotide sequence encoding a foreign gene further downstream, and a nucleotide sequence containing a second inverted terminal repeat (ITR) or a functional equivalent thereof further downstream.

In one embodiment of the present invention, the foreign gene contained in the rAAV genome and the AAV vector encodes a protein capable of exhibiting a physiological activity in vivo. Such a protein having a physiological activity (physiologically active protein) is not particularly limited, and examples of a preferred protein include proteins that should be administered to a patient over a long period of time as a drug. Examples of such a drug include a growth hormone, a lysosomal enzyme, somatomedin, insulin, glucagon, a cytokine, a lymphokine, a blood clotting factor, an antibody, a fusion protein of an antibody and another protein, granulocyte-macrophage colony-stimulating factor (GM-CSF), granulocyte colony-stimulating factor (G-CSF), macrophage colony-stimulating factor (M-CSF), erythropoietin, darbepoetin, tissue plasminogen activator (t-PA), thrombomodulin, follicle-stimulating hormone (FSH), gonadotropic hormone-releasing hormone (GnRH), gonadotropin, DNasel, thyroid-stimulating hormone (TSH), nerve growth factor (NGF), ciliary neurotrophic factor (CNTF), glial cell line-derived neurotrophic factor (GDNF), neurotrophin 3, neurotrophin 4/5, neurotrophin 6, neuregulin 1, activin, basic fibroblast growth factor (bFGF), fibroblast growth factor 2 (FGF2), epithelial growth factor (EGF), vascular endothelial growth factor (VEGF), interferon α, interferon β, interferon γ, interleukin 6, PD-1, a PD-1 ligand, a tumor necrosis factor α receptor (TNF-α receptor), an enzyme having activity for degrading beta-amyloid, etanercept, pegvisomant, metreleptin, abatacept, asfotase, a GLP-1 receptor agonist, and any one of antibody drugs.

Preferred examples when the physiologically active protein is a lysosomal enzyme include α-L-iduronidase, iduronate-2-sulfatase, glucocerebrosidase, β-galactosidase, a GM2-activator protein, β-hexosaminidase A, β-hexosaminidase B, N-acetyl-glucosamine-1-phosphotransferase, α-mannosidase, β-mannosidase, galactosylceramidase, saposin C, arylsulfatase A, α-L-fucosidase, aspartylglucosaminidase, α-N-acetyl-galactosaminidase, acidic sphingomyelinase, α-galactosidase A, β-glucuronidase, heparin-N-sulfatase, α-N-acetyl-glucosaminidase, acetyl CoAα-glucosaminide N-acetyl transferase, N-acetyl glucosamine-6-sulfate sulfatase, acid ceramidase, amylo-1,6-glucosidase, sialidase, palmitoyl protein thioesterase-1, tripeptidyl peptidase-1, hyaluronidase-1, CLN1, and CLN2.

In one embodiment of the present invention, the physiologically active protein is a human protein. In addition, the protein may be a wild-type protein, or may be a mutated protein as long as the protein has the original physiological activity. Here, the fact that the protein has the original physiological activity means that the protein has a physiological activity of 20% or more with respect to the physiological activity of a wild-type protein of the protein. The physiological activity of the protein with respect to the physiological activity of the wild-type protein is more preferably 40% or more, still more preferably 50% or more, still more preferably 80% or more, and further still more preferably 90% or more.

In a case where amino acids of an amino acid sequence of a wild-type physiologically active protein are substituted with other amino acids, the number of amino acids to be substituted is preferably 1 to 20, more preferably 1 to 10, and still more preferably 1 to 3. In a case where the amino acid sequence of the wild-type protein is deleted, the number of amino acids to be deleted is preferably 1 to 20, more preferably 1 to 10, and still more preferably 1 to 3. In addition, a mutation combining substitution and deletion of these amino acids can also be added. In a case where an amino acid is added to the wild-type protein, preferably 1 to 20 amino acids, more preferably 1 to 10 amino acids, and still more preferably 1 to 3 amino acids are added in the amino acid sequence of the protein or to the 5' terminal or the 3' terminal. A mutation combining addition, substitution, and deletion of these amino acids can also be added. The amino acid sequence of the protein to which the mutation is added shows preferably 80% or more identity, more preferably 85% or more identity, still more preferably 90% or more identity, still more preferably 95% or more identity, and further still more preferably 98% or more identity with the amino acid sequence of the wild-type protein.

Note that, in the present invention, the position and form thereof (deletion, substitution, or addition) of each mutation as compared with the wild-type protein can be easily confirmed by aligning the amino acid sequences of the wild-type and mutant proteins. Note that, in the present invention, the identity between the amino acid sequence of the wild-type protein and the amino acid sequence of the mutant protein can be easily calculated using a well-known homology calculation algorithm. Examples of such an algorithm include BLAST (Altschul SF. J Mol. Biol. 215. 403-10, (1990)), a similarity search method of Pearson and Lipman (Proc. Natl. Acad. Sci. USA. 85. 2444 (1988)), a local homology algorithm of Smith and Waterman (Adv. Appl. Math. 2. 482-9 (1981)).

Substitution of an amino acid in the amino acid sequence of the protein with another amino acid occurs, for example, within an amino acid family that is related thereto in side chains and chemical properties of the amino acids. It is predicted that such substitution within the amino acid family does not significantly change the function of the protein (that is, a conservative amino acid substitution). Such an amino acid family include, for example:
(1) aspartic acid and glutamic acid which are acidic amino acids;
(2) histidine, lysine, and arginine which are basic amino acids;
(3) phenylalanine, tyrosine, and tryptophan which are aromatic amine acids;
(4) serine and threonine which are amino acids having a hydroxyl group (hydroxyamino acids);
(5) methionine, alanine, valine, leucine, and isoleucine which are hydrophobic amino acids;
(6) cysteine, serine, threonine, asparagine, and glutamine which are neutral hydrophilic amino acids;
(7) glycine and proline which are amino acids affecting an orientation of a peptide chain;
(8) asparagine and glutamine which are amide type amino acids (polar amino acids);
(9) alanine, leucine, isoleucine, and valine which are aliphatic amino acids;
(10) alanine, glycine, serine, and threonine which are amino acids having a small side chain; and
(11) alanine and glycine which are particularly small amino acids of a side chain.

In addition, a fusion protein of the physiologically active protein and an antibody is also one of preferred proteins that can exhibit a physiological activity. Here, the antibody to be fused with a physiologically active protein is not particularly limited as long as it exhibits an affinity for a specific antigen in vivo. For example, the antibody is an antibody having a specific affinity for a protein present on a surface of a vascular endothelial cell. Examples of the protein present on the surface of the vascular endothelial cell include a transferrin receptor, an insulin receptor, a leptin receptor, an insulin-like growth factor I receptor, an insulin-like growth factor II receptor, a lipoprotein receptor, an Fc Receptor, a glucose transporter 1, an organic anion transporter, a monocarboxylic acid transporter, a low-density lipoprotein receptor-related protein 1, a low-density lipoprotein receptor-related protein 8, and a membrane-bound precursor of a heparin-binding epidermal growth factor-like growth factor. Furthermore, examples of the organic anion transporter include OATP-F, and examples of the monocarboxylic acid transporter include MCT-8. Antibodies having a specific affinity for the transferrin receptor and the insulin receptor can be preferably used, and in particular, antibodies having a specific affinity for the transferrin receptor can be preferably used. In a case where the antibody binds to a protein present on the surface of the vascular endothelial cell, the fusion protein can reach various tissues after being taken up by the vascular endothelial cell and exhibit a physiological activity there. Therefore, such a fusion protein can be used as a drug that should exhibit drug efficacy in various tissues. For example, in a case where the antibody has a specific affinity for the transferrin receptor and the insulin receptor, such a fusion protein can be used as a drug that should exert drug efficacy in muscle.

In one embodiment of the present invention, the antibody to be fused with a physiologically active protein is an antibody having a specific affinity for a protein present on a surface of a cerebral vascular endothelial cell. Examples of the protein present on the surface of the cerebral vascular endothelial cell include a transferrin receptor, an insulin receptor, a leptin receptor, an insulin-like growth factor I receptor, an insulin-like growth factor II receptor, a lipoprotein receptor, an Fc Receptor, a glucose transporter 1, an organic anion transporter, a monocarboxylic acid transporter, a low-density lipoprotein receptor-related protein 1, a low-density lipoprotein receptor-related protein 8, and a membrane-bound precursor of a heparin-binding epidermal growth factor-like growth factor. Furthermore, examples of the organic anion transporter include OATP-F, and examples of the monocarboxylic acid transporter include MCT-8. Antibodies having a specific affinity for the transferrin receptor and the insulin receptor can be preferably used, and in particular, antibodies having a specific affinity for the transferrin receptor can be preferably used. In a case where the antibody binds to a protein present on the surface of the cerebral vascular endothelial cell, the fusion protein can reach brain tissues after passing through a blood-brain barrier (BBB) and exhibit a physiological activity there. Therefore, such a fusion protein can be used as a drug that should exhibit drug efficacy in the brain. Instead of the antibody fused with the physiologically active protein, other ligands having a specific affinity for proteins present on the surface of the cerebral vascular endothelial cell may be fused with the physiologically active protein. Examples of such ligands include transferrin, insulin, an insulin-like growth factor I, an insulin-like growth factor II receptor, a lipoprotein, and an Fc fragment, but such ligands are not limited thereto.

In one embodiment of the present invention, a nucleotide sequence that can be used as a nucleotide sequence containing a gene expression control site for controlling expression of a gene of a foreign protein is not particularly limited as long as it can express a foreign protein in a cell, tissue, or organism of a mammal (particularly a human) into which a gene encoding the foreign protein is introduced, and it is preferable that the nucleotide sequence contains a cytomegalovirus-derived promoter (optionally containing an enhancer), a SV40 early promoter, a human elongation factor-1α (EF-1α) promoter, a human ubiquitin C promoter, a retroviral Rous sarcoma virus LTR promoter, a dihydrofolate reductase promoter, a β-actin promoter, a phosphoglycerate kinase (PGK) promoter, a mouse albumin promoter, a human promoter, and a human α-1 antitrypsin promoter. For example, a synthetic promoter (mouse α-fetoprotein enhancer/mouse albumin promoter) containing a nucleotide sequence containing a mouse albumin promoter downstream of the mouse α-fetoprotein enhancer can be preferably used as a gene expression control site.

The gene control site may be a promoter of a gene expressed in an organ-specific manner or a cell type-specific manner. By using an organ-specific expression promoter or a cell type-specific expression promoter, a gene encoding a foreign protein incorporated into an rAAV genome can be specifically expressed in a desired organ or cell.

Hereinafter, one embodiment of a production method of rAAV9 virions in the present invention will be described in detail. First, mammalian cells to be host cells are cultured. As the host cell, a mammalian cell in which an E1A gene and an E1B gene of adenovirus are incorporated into a genome is used. HEK293T cells, which are cells derived from human embryonic kidney tissues, are preferred examples of such cells. The amount of host cells to be cultured is appropriately prepared according to the amount of rAAV9 virions required to be produced.

Next, a plasmid containing an AAV vector is introduced into the host cell. The introduced plasmid is usually three kinds of plasmids such as (1) a plasmid having a structure containing a nucleotide sequence containing a first inverted terminal repeat (ITR) and a nucleotide sequence containing a second inverted terminal repeat (ITR) derived from a virus such as AAV, and a gene encoding a desired protein located between the two ITRs (plasmid 1), (2) a plasmid containing an AAV Rep gene having a function required for incorporating the nucleotide sequence of a region (including an ITR sequence) sandwiched by the ITR sequences into a genome of a host cell and a gene encoding an AAV capsid protein (plasmid 2), and (3) a plasmid containing an E2A region, an E4 region, and a VA1 RNA region of adenovirus (plasmid 3). However, the present invention is not limited thereto, and one plasmid obtained by linking two plasmids among the plasmids 1 to 3 can be used, and two kinds of plasmids of the remaining one plasmid can also be used. Furthermore, one plasmid obtained by linking the plasmids 1 to 3 can also be used. The plasmid 2 contains a gene encoding an AAV9 capsid protein. The introduction of the plasmid into the host cell can be performed by a conventional method including electroporation, a liposome-mediated method, and the like. A method using lipofectamine can be preferably used for introduction of a plasmid into a host cell.

After introduction of the plasmid, the host cells are cultured. During this culture period, the rAAV genome is replicated in the host cell, and this rAAV genome is packaged in the AAV9 capsid protein, such that rAAV9 virions are formed and released into a medium. At this time, the medium used for culturing the host cells is not particularly limited as long as it is a medium that can be used for culturing the host cells, and for example, Eagle's minimum essential medium containing 10% FBS is one of media that can be preferably used. After introduction of the plasmid, the host cells are preferably cultured for 3 to 8 days, for example, 6 days.

After completion of the culture, a culture supernatant containing the rAAV9 virions is recovered. The recovery of the culture supernatant can be performed by a conventional method such as a centrifugal separation method or a membrane filtration method for the medium after completion of the culture. DNA derived from the genome of the host cell or the like may be attached to the surface of the rAAV9 virion contained in the culture supernatant. The DNA attached to the rAAV9 virion can be degraded by adding a DNA degrading enzyme to the recovered culture supernatant. As the DNA degrading enzyme, an endonuclease can be preferably used. An endonuclease derived from Serratia marcescens is an example thereof, and benzonase, which is a variant of this endonuclease, is one of those that can be preferably used. The culture supernatant treated with the DNA degrading enzyme is subjected to a purification process described below in detail. Note that the purification process includes affinity column chromatography as a first step and anion exchange column chromatography as a second step, and the purification process can include anion exchange column chromatography as a first step and affinity column chromatography as a second step. In addition, in addition to these types of column chromatography, a purification process including one or a plurality of types of column chromatography using a carrier selected from a cation exchange carrier, an anion exchange carrier, a hydrophobic interaction carrier, a size exclusion carrier, a reverse phase carrier, a hydroxyapatite carrier, a fluorapatite carrier, and a mixed mode carrier can be performed. Hereinafter, a purification process including affinity column chromatography as a first step and anion exchange column chromatography as a second step will be described in detail.

The first step of the purification process is affinity column chromatography using a column in which a material having an affinity for the AAV9 capsid is immobilized on a carrier. The material having an affinity for the AAV9 capsid used at this time is not particularly limited.

The material having an affinity for the AAV9 capsid immobilized on the carrier in the affinity column chromatography is not particularly limited as long as the material has a specific affinity for the AAV9 capsid, and a dissociation constant between the material and the AAV9 capsid is preferably 1 × 10⁻⁶ M or less, for example, 5 × 10⁻⁷ M to 1 × 10⁻⁹ M. Examples of such a material include anti-AAV9 capsid antibodies. The dissociation constant of the anti-AAV9 capsid antibody used here with the AAV9 capsid is preferably 1 × 10⁻⁶ M or less, for example, 5 × 10⁻⁷ M to 1 × 10⁻⁹ M. POROS^{™} CaptureSelect^{™} AAVX Resins (Thermo Fisher Scientific Inc.) are preferred examples of the material that can be used for affinity column chromatography.

In a case where the carrier for affinity column chromatography is a carrier on which an anti-AAV9 capsid antibody is immobilized (antibody column chromatography), the column is equilibrated in advance with a buffer solution near neutrality containing a neutral salt. The neutral salt used here is not particularly limited, but NaCl is preferably used. A concentration of the neutral salt is preferably 100 to 200 mM, and more preferably 130 to 170 mM, for example, 150 mM. A pH of the buffer solution is preferably adjusted to 7.0 to 8.0 mM, and more preferably 7.2 to 7.7 mM, for example, 7.5 mM. A buffer solution to which MgCl₂ is further added can also be used, and a concentration thereof is preferably 2 to 6 mM, for example, 4 mM.

The rAAV9 virions contained in the culture supernatant bind to the column by loading the culture supernatant onto the column, and then the column is washed, such that most of impurities can be removed. However, it is hardly possible to remove the empty capsid in this step. This is because the empty capsid also binds to the column.

The rAAV9 virions adsorbed on the carrier in the antibody column chromatography can be eluted by passing an acidic buffer solution containing a buffer through the column. The buffer used at this time is not particularly limited, and preferred examples thereof include citric acid, phosphoric acid, glycine, histidine, and acetic acid. A concentration of the buffer is preferably 10 mM to 100 mM, for example, 50 mM. A pH of the buffer solution is preferably adjusted to 3.0 to 4.0 mM, and more preferably 3.2 to 3.7 mM, for example, 3.5 mM. A buffer solution to which MgCl₂ is further added can also be used, and a concentration thereof is preferably 2 to 6 mM, for example, 4 mM. This eluate also contains empty capsids in addition to the rAAV9 virions.

A pH of a solution containing rAAV9 virions eluted from antibody column chromatography is acidic. The pH of this solution is adjusted to basic pH using a buffer solution after elution. The buffer contained in the buffer solution used at this time is not particularly limited, tris-hydrochloric acid, Bis-Tris, Bis-Tris propane, bicine (N,N-bis(2-hydroxyethyl)glycine), 4-2-hydroxyethyl-1-piperazineethanesulfonic acid (HEPES), 3-{[tris(hydroxymethyl)methyl]amino}propane ethanesulfonic acid (TAPS), tricine (N-tris(hydroxymethyl)methylglycine), and the like can be used, and for example, Bis-Tris propane is one of the preferred buffers. The concentration of the buffer is not particularly limited as long as the buffer solution has a buffering capacity, and is preferably 5 mM to 80 mM, more preferably 10 mM to 60 mM, and still more preferably 10 mM to 30 mM, for example, 20 mM. The pH of the buffer solution is not particularly limited as long as it is basic. In addition, the buffer solution may contain a neutral salt in addition to the buffer. Examples of the neutral salt contained in the buffer solution at this time include calcium chloride, magnesium chloride, potassium phosphate, and sodium chloride, and in particular, magnesium chloride is used. A concentration of the neutral salt is not particularly limited, and is preferably 0.1 mM to 20 mM, more preferably 0.5 mM to 10 mM, and still more preferably 1 mM to 10 mM, for example, 2 mM. The buffer solution may further contain a nonionic surfactant. As the nonionic surfactant, a polyoxyethylene-polyoxypropylene block copolymer, a polysorbate, and a poloxamer can be preferably used, and for example, polysorbate 20, polysorbate 80, and poloxamer 188 can be particularly preferably used. A concentration of the nonionic surfactant is preferably 0.0002 to 0.01% (w/v), more preferably 0.0005 to 0.005% (w/v), and still more preferably 0.001 to 0.002% (w/v), for example, 0.001% (w/v). Examples of a preferred buffer solution include a 20 mM Bis-Tris propane buffer solution containing 4 mM MgCl₂ and 0.001% (w/v) Pluronic^{™} F-68 (Poloxamer 188). A pH of an eluate after addition of the buffer is preferably pH 8.5 to 10.5, and more preferably pH 9.0 to 10.0, for example, pH 9.5.

The second step of the purification process is anion exchange column chromatography using a material containing an anion exchange group as a stationary phase. This step is for separating the rAAV9 virions and the empty capsids by performing gradient elution or stepwise elution depending on a difference in conductivity of the eluate by using an anion exchange column. The stationary phase used for the anion exchange column chromatography is preferably a strong ion exchanger. For example, strong anion exchange column chromatography using a strong anion exchanger containing a quaternary amine as a stationary phase can be preferably used.

In the anion exchange column chromatography, the anion exchange column is pre-equilibrated with a buffer solution before a basic pH-adjusted solution containing rAAV9 virions and empty capsids is loaded. The buffer contained in the buffer solution used at this time is not particularly limited, tris-hydrochloric acid, Bis-Tris, Bis-Tris propane, bicine (N,N-bis(2-hydroxyethyl)glycine), 4-2-hydroxyethyl-1-piperazineethanesulfonic acid (HEPES), 3-{[tris(hydroxymethyl)methyl]amino}propane ethanesulfonic acid (TAPS), tricine (N-tris(hydroxymethyl)methylglycine), and the like can be used, and for example, Bis-Tris propane is one of the preferred buffers. The concentration of the buffer is not particularly limited as long as the buffer solution has a buffering capacity, and is preferably 5 mM to 80 mM, more preferably 10 mM to 60 mM, and still more preferably 10 mM to 30 mM, for example, 20 mM. A pH of the buffer solution is not particularly limited as long as it is basic, and is preferably pH 8.5 to 10.5, and more preferably 9.0 to 10.0, for example, pH 9.5. In addition, the buffer solution may contain a neutral salt in addition to the buffer, examples of the neutral salt contained at this time include calcium chloride, magnesium chloride, potassium phosphate, and sodium chloride, and magnesium chloride is preferable. A concentration of the neutral salt is not particularly limited, and is preferably 0.1 mM to 20 mM, more preferably 0.5 mM to 10 mM, and still more preferably 1 mM to 10 mM, for example, 2 mM. The buffer solution may further contain a nonionic surfactant. As the nonionic surfactant, a polyoxyethylene-polyoxypropylene block copolymer, a polysorbate, and a poloxamer can be preferably used, and for example, polysorbate 20, polysorbate 80, and poloxamer 188 can be particularly preferably used. A concentration of the nonionic surfactant is preferably 0.0002 to 0.01% (w/v), more preferably 0.0005 to 0.005% (w/v), and still more preferably 0.001 to 0.002% (w/v), for example, 0.001% (w/v). Examples of a preferred buffer solution include a 20 mM Bis-Tris propane buffer solution containing 4 mM MgCl₂ and 0.001% (w/v) Pluronic^{™} F-68 (Poloxamer 188).

In the anion exchange column chromatography, rAAV9 virions adsorbed on the column can be eluted from the column using a basic buffer solution (eluate) containing a constant concentration of a quaternary ammonium salt or by continuously changing the concentration of the quaternary ammonium salt in the basic buffer solution, and are recovered by fractionating fractions containing the rAAV9 virions. As one of the functions of the anion exchange column chromatography, the rAAV9 virions and the empty capsids may be separated. Since the rAAV9 virion is packaged with the rAAV9 genome, the rAAV9 virion is more negatively charged than the empty capsid. Thus, the rAAV9 virion is adsorbed strongly on anion exchange column chromatography under a basic environment compared to the empty capsid. Therefore, when a buffer solution containing a quaternary ammonium salt at a certain concentration or higher is passed through the anion exchange column chromatography on which the rAAV9 virions and the empty capsids are adsorbed, generally, the empty capsids are eluted first, and then the rAAV9 virions are eluted.

The buffer contained in the eluate used to elute the rAAV9 virions adsorbed on the column in the anion exchange column chromatography is not particularly limited, tris-hydrochloric acid, Bis-Tris, Bis-Tris propane, bicine (N,N-bis(2-hydroxyethyl)glycine), 4-2-hydroxyethyl-1-piperazineethanesulfonic acid (HEPES), 3-{[tris(hydroxymethyl)methyl]amino}propane ethanesulfonic acid (TAPS), tricine (N-tris(hydroxymethyl)methylglycine), and the like can be used, and for example, Bis-Tris propane is one of the preferred buffers. A concentration of the buffer contained in the eluate is not particularly limited as long as the buffer solution has a buffering capacity, and is preferably 5 mM to 80 mM, more preferably 10 mM to 60 mM, and still more preferably 10 mM to 30 mM, for example, 20 mM. A pH of the buffer solution is not particularly limited as long as it is basic, and is preferably pH 8.5 to 10.5, and more preferably 9.0 to 10.0, for example, pH 9.5.

In a case where the rAAV9 virions are eluted by a gradient elution method in which a concentration of the quaternary ammonium salt contained in the eluate is continuously changed, a range of the concentration is preferably set to 0 mM to 200 mM, for example, 0 mM to 80 mM, 0 mM to 60 mM, 0 mM to 50 mM, 10 mM to 80 mM, 20 mM to 80 mM, 20 mM to 50 mM, 20 mM to 60 mM, or the like. The eluate eluted from the column is continuously monitored for an absorbance at 260 nm or/and 280 nm and recovered for each peak appearing in the column chromatogram. Among these fractions, a fraction having a ratio of the rAAV9 virions and the empty capsids of a desired value or more is recovered. The ratio of the rAAV9 virions and the empty capsids is arbitrarily set depending on the application of the rAAV9 virions, and is preferably 2 : 8 to 10 : 0, more preferably 4 : 6 to 10 : 0, and still more preferably 5 : 5 to 10 : 0, and can be, for example, 7 : 3 to 10 : 0 or 8 : 2 to 10 : 0.

In a case where the rAAV9 virions are eluted using an eluate containing a constant concentration of a quaternary ammonium salt, the concentration thereof is preferably 20 mM to 60 mM, and more preferably 25 mM to 55 mM or 30 mM to 55 mM, and is set to, for example, 30 mM, 35 mM, 40 mM, 44 mM, 48 mM, or the like. The eluate eluted from the column is continuously monitored for an absorbance at 260 nm or/and 280 nm and recovered for each peak appearing in the column chromatogram. Among these fractions, a fraction having a ratio of the rAAV9 virions and the empty capsids of a desired value or more is recovered. The ratio of the rAAV9 virions and the empty capsids is arbitrarily set depending on the application of the rAAV9 virions, and is preferably 2 : 8 to 10 : 0, more preferably 4 : 6 to 10 : 0, and still more preferably 5 : 5 to 10 : 0, for example, 7 : 3 to 10 : 0 or 8 : 2 to 10 : 0. Note that even in a case where the concentration of the quaternary ammonium salt increases stepwise to elute the rAAV9 virions, when the rAAV9 virions are eluted at a certain concentration that increases stepwise, the quaternary ammonium salt is contained in a case where the rAAV9 virions are eluted using an eluate containing a quaternary ammonium salt at a certain concentration.

In addition, the eluate may contain a neutral salt in addition to the buffer and the quaternary ammonium salt, examples of the neutral salt contained at this time include calcium chloride, magnesium chloride, potassium phosphate, and sodium chloride, and magnesium chloride is preferable. A concentration of the neutral salt is not particularly limited, and is preferably 0.1 mM to 20 mM, more preferably 0.5 mM to 10 mM, and still more preferably 1 mM to 10 mM, for example, 2 mM. The buffer solution may further contain a nonionic surfactant. As the nonionic surfactant, a polyoxyethylene-polyoxypropylene block copolymer, a polysorbate, and a poloxamer can be preferably used, and for example, polysorbate 20, polysorbate 80, and poloxamer 188 can be particularly preferably used. A concentration of the nonionic surfactant is preferably 0.0002 to 0.01% (w/v), more preferably 0.0005 to 0.005% (w/v), and still more preferably 0.001 to 0.002% (w/v), for example, 0.001% (w/v). Examples of a preferred buffer solution include a 20 mM Bis-Tris propane buffer solution containing 4 mM MgCl₂, 0.001% (w/v) Pluronic^{™} F-68 (poloxamer 188), and a quaternary ammonium salt at a concentration of 30 mM, 35 mM, 40 mM, 44 mM, or 48 mM.

In one embodiment in anion exchange column chromatography, the rAAV9 virions are eluted using an eluate containing a quaternary ammonium salt at a constant concentration of 30 mM to 60 mM, for example, at a constant concentration of 30 mM, 35 mM, 40 mM, 44 mM, or 48 mM, such that a chromatogram in which a peak having a large proportion of empty capsids and a peak having a large proportion of rAAV9 virions appear separately in this order can be obtained. rAAV9 virions having fewer empty capsids can be obtained by fractionating only the fraction corresponding to the peak having a large proportion of rAAV9 virions. A ratio of the rAAV9 virions and the empty capsids in the eluted fraction recovered in this case is preferably 2 : 8 to 10 : 0, more preferably 4 : 6 to 10 : 0, and still more preferably 5 : 5 to 10 : 0, and can be arbitrarily adjusted to 5 : 5 to 10 : 0, 6 : 4 to 10 : 0, 7 : 3 to 10 : 0, 8 : 2 to 10 : 0, or the like. Note that the ratio of the empty capsids and the rAAV9 virions can be measured, for example, by the method described in Example 15.

In the present specification, a solution containing the rAAV9 virions obtained by the production method of the present invention is referred to as an rAAV9 virion purified product. The rAAV9 virion purified product has less contamination with empty capsids, and a ratio of the rAAV9 virions and the empty capsids is arbitrarily set. For example, the ratio is 5 : 5 to 10 : 0, 6 : 4 to 10 : 0, 7 : 3 to 10 : 0, 8 : 2 to 10 : 0, or the like.

The rAAV9 virion purified product can be aseptically packaged in a glass container, a resin container, or the like to be used as a pharmaceutical composition as a therapeutic agent. The rAAV9 virion purified product can also be further subjected to a purification process for the purpose of removing endotoxin or the like.

The production method of rAAV9 virions of the present invention can be preferably used as a production method of rAAV9 virions on a commercial scale since scale-up can be easily performed as a method for separating the rAAV9 virions and the empty capsids.

### Examples

Hereinafter, the present invention will be described in more detail with reference to Examples, but the present invention is not intended to be limited to Examples.

### [Example 1] Construction of pHelper(mod) Vector

A DNA fragment containing a nucleotide sequence set forth in SEQ ID NO: 1 containing an SalI site, an RsrII site, a replication origin (ColE1 ori), an ampicillin resistance gene, a BsiWI site, a BstZ17I site, and a BsrGI site from the 5' side was synthesized. The DNA fragment was digested with SalI and BsrGI. The pHelper vector (Takara Bio Inc.) was digested with SalI and BsrGI, and the restriction enzyme-treated DNA fragment was inserted into the digested pHelper vector. The obtained plasmid was used as a pHelper(mod) vector (FIG. 1).

### [Example 2] Construction of pAAV-CBA(BAM)-PPT1-WPRE Vector

A DNA fragment containing a nucleotide sequence set forth in SEQ ID NO: 2 containing a ClaI site, a human CMV enhancer, a chicken β-actin promoter, a chicken β-actin/MVM chimeric intron, a palmitoyl-protein thioesterase-1 (PPT1) gene, a woodchuck hepatitis virus posttranscriptional regulatory element (WPRE), a bovine growth hormone polyA sequence, and a BglII site from the 5' side was synthesized. The DNA fragment was digested with ClaI and BglII. The pAAV-CMV vector (Takara Bio Inc.) was digested with ClaI and BglII, and the restriction enzyme-treated DNA fragment was inserted into the digested pAAV-CMV vector. The obtained plasmid was a pAAV-CBA(BAM)-PPT1-WPRE vector (FIG. 2). The plasmid was purified by a conventional method.

### [Example 3] Construction of pR2(mod)C6 Vector

A DNA fragment containing a nucleotide sequence set forth in SEQ ID NO: 3 containing an AfeI site, an RsrII site, a BsrGI site, an AvrII site, an ampicillin resistance gene site, a replication origin (ColE1 ori), an RsrII site, a 5' portion of an AAV2 Rep region (containing a p5 promoter), and an SacII site from the 5' side was synthesized The DNA fragment was digested with AfeI and SacII. The pRC6 vector (Takara Bio Inc.) was digested with AfeI and SacII, and the restricted synthetic gene was inserted into the digested pRC6 vector. The obtained plasmid was used as a pR2(mod)C6 vector (FIG. 3). The plasmid was purified by a conventional method.

### [Example 4] Construction of pR2(mod)C9 Vector

A DNA fragment containing a nucleotide sequence set forth in SEQ ID NO: 4 containing a HindIII site, a 3' portion of an AAV2 Rep region, an AAV9 Cap region, a p5 promoter, an RsrII site, and a BsrGI site from the 5' side was synthesized. The DNA fragment was digested with HindIII and BsrGI. The pR2(mod)C6 vector was digested with HindIII and BsrGI, and the restriction enzyme-treated synthetic gene was inserted into the digested pR2(mod)C6 vector. The obtained plasmid was used as a pR2(mod)C9 vector (FIG. 4). The plasmid was purified by a conventional method.

### [Example 5] Production of rAAV9 Virions

Fetal bovine serum (FBS, Collected in South America, CAPRICORN SCIENTIFIC GmbH) was added to Eagle's minimum essential medium (Minimum Essential Medium Eagle With Earle's salts, L-glutamine and sodium bicarbonate, liquid, sterile-filtered, suitable for cell culture, MERCK Corporation) so that a final concentration was 10%, thereby obtaining a 10% FBS-containing MEM medium. HEK293T cells as a cell line derived from human embryonic kidney cells expressing a large T antigen gene of SV40 virus were expansively cultured in the 10% FBS-containing MEM medium until the number of cells reached approximately 6.5 × 10⁹. HEK293T cells were suspended in 1,000 mL of the 10% FBS-containing MEM medium so that a cell concentration was 6.3 × 10⁵ cells/mL, and the suspended HEK293T cells were seeded in a cell stack culture chamber (cell culture surface treatment, 10 chambers, Corning Inc.) and cultured at 37°C in the presence of 5% CO₂ for 16 hours.

1.1 mg of the pHelper(mod) vector, 0.55 mg of the pR2(mod) C9 vector, 0.55 mg of the pAAV-CBA(BAM)-PPT1-WPRE vector, and 4.4 mg of polyethyleneimine (PEI MAX-Transfection Grade Linear Polyethylenimine Hydrochloride (MW 40,000)) were added to Eagle's minimum essential medium so that a liquid volume was 54 mL, stirring was performed with a vortex mixer, and the mixture was allowed to stand at room temperature for 15 minutes. 1,446 mL of Eagle's minimum essential medium was added thereto so that a total amount was 1,500 mL, and the resulting product was used as a transfection reagent.

The entire medium was removed from the cell stack culture chamber after the culture for 16 hours, the total amount of 1,500 mL of the transfection reagent was added thereto, and then the cells were cultured at 37°C in the presence of 5% CO₂.

### [Example 6] Cell Removal and Nuclease Treatment

On day 6 of culture, the medium was recovered from the cell stack culture chamber, and filtered with a depth filter for prefiltration (ULTA Prime GF 5.0 µm, 2 inch capsule, GG, Cytiva) and a bottle top filter (Nalgene^{™} Rapid-Flow^{™} Sterile Single Use Bottle Top Filters, Thermo Fisher Scientific Inc.) to recover a culture supernatant. 10 U/mL of Benzonase^{™} (Benzonase Nuclease, Purity > 90%,Merck Corporation) was added to the culture supernatant, and stirring was performed at 37°C for 1 hour for reaction. In this reaction, DNA fragments present in the filtrate, for example, DNA fragments attached to rAAV virions present in the filtrate are degraded by endonuclease activity of Benzonase^{™}. The solution after the reaction was filtered using a filter of the same type as the bottle top filter described above, and the filtrate was subjected to the following process as an rAAV9 virion-containing enzyme treatment solution.

### [Example 7] Purification Process by Affinity Column Chromatography

A 20 mM trishydroxymethylaminomethane buffer solution (pH 7.5) containing 4 mM MgCl₂ and 150 mM NaCl having a volume 5 times larger than a column volume was passed through a column (diameter of 1.6 cm and bed height of 10 cm) filled with 20 mL of POROS AAVX resin (POROS^{™} CaptureSelect^{™} AAVX Affinity Resin, Thermo Fisher Scientific Inc.) at a flow rate of 5 mL/min to equilibrate the column. Next, the rAAV9 virion-containing enzyme treatment solution obtained in Example 6 was loaded onto the column at a flow rate of 5 mL/min. Next, a 20 mM trishydroxymethylaminomethane buffer solution (pH 7.5) containing 4 mM MgCl₂ and 150 mM NaCl having a volume 5 times larger than the column volume was passed through the column at the same flow rate, the column was washed, and then a 50 mM citric acid buffer solution (pH 3.5) containing 4 mM MgCl₂ having a volume 3 times larger than the column volume was passed through the column at the same flow rate to elute rAAV9 virions adsorbed on the column. A 20 mM Bis-Tris Propane buffer solution (pH 10.6) containing 2 mM MgCl₂ was added to the obtained eluate to adjust the pH to 9.5. The eluate was used as an rAAV9 virion-containing affinity fraction, and was subjected to a purification process by anion exchange column chromatography under the following conditions A to F.

### [Example 8] Purification Process by Anion Exchange Column Chromatography (Condition A)

A 20 mM Bis-Tris Propane buffer solution (pH 9.5) containing 4 mM MgCl₂ and 0.001% Pluronic^{™} F-68 having a volume 8 times larger than a column volume was passed through a column (diameter of 0.8 cm and bed height of 20 cm) filled with 10 mL of TSKgel SuperQ-5PW(20) resin (TOSOH CORPORATION) at a flow rate of 1.25 mL to equilibrate the column. Next, 270 mL of the obtained rAAV9 virion-containing affinity fraction obtained in Example 7 was loaded onto the column at a flow rate of 1.25 mL/min. Next, a 20 mM Bis-Tris Propane buffer solution (pH 9.5) containing 4 mM MgCl₂ and 0.001% Pluronic^{™} F-68 having a volume 5 times larger than the column volume was passed through the column to wash the column. Next, a 20 mM Bis-Tris Propane buffer solution (pH 9.5) containing tetramethylammonium chloride, 4 mM MgCl₂, and 0.001% Pluronic^{™} F-68 having a volume 100 times larger than the column volume was passed through the column at a flow rate of 1.25 mL/min while increasing the concentration of tetramethylammonium chloride at a linear gradient (0 to 200 mM) to elute the rAAV9 virions from the column. At this time, an absorptiometer was installed in a flow path downstream of a discharge port of the column, and absorbances at 260 nm and 280 nm of the eluate were continuously monitored to obtain a chromatogram. The eluate was recovered for each fraction corresponding to a peak on the chromatogram.

### [Example 9] Purification Process by Anion Exchange Column Chromatography (Condition B)

A 20 mM Bis-Tris Propane buffer solution (pH 9.5) containing 4 mM MgCl₂ and 0.001% Pluronic^{™} F-68 having a volume 8 times larger than a column volume was passed through a column (diameter of 0.8 cm and bed height of 20 cm) filled with 10 mL of TSKgel SuperQ-5PW(20) resin (TOSOH CORPORATION) at a flow rate of 1.25 mL to equilibrate the column. Next, 270 mL of the obtained rAAV9 virion-containing affinity fraction obtained in Example 7 was loaded onto the column at a flow rate of 1.25 mL/min. Next, a 20 mM Bis-Tris Propane buffer solution (pH 9.5) containing 4 mM MgCl₂ and 0.001% Pluronic^{™} F-68 having a volume 5 times larger than the column volume was passed through the column to wash the column. Next, a 20 mM Bis-Tris Propane buffer solution (pH 9.5) containing 4 mM MgCl₂, 0.001% Pluronic^{™} F-68, and 30 mM tetramethylammonium chloride having a volume 10 times larger than the column volume was passed through the column at a flow rate of 1.25 mL/min to elute the rAAV9 virions from the column. Furthermore, a 20 mM Bis-Tris Propane buffer solution (pH 9.5) containing 4 mM MgCl₂, 0.001% Pluronic^{™} F-68, and 48 mM tetramethylammonium chloride having a volume 6 times larger than the column volume was passed through the column at a flow rate of 1.25 mL/min to elute the rAAV9 virions from the column. At this time, an absorptiometer was installed in a flow path downstream of a discharge port of the column, and absorbances at 260 nm and 280 nm of the eluate were continuously monitored to obtain a chromatogram. The eluate was recovered for each fraction corresponding to a peak on the chromatogram.

### [Example 10] Purification Process by Anion Exchange Column Chromatography (Condition C)

A 20 mM Bis-Tris Propane buffer solution (pH 9.5) containing 4 mM MgCl₂ and 0.001% Pluronic^{™} F-68 having a volume 8 times larger than a column volume was passed through a column (diameter of 0.8 cm and bed height of 20 cm) filled with 10 mL of TSKgel SuperQ-5PW(20) resin (TOSOH CORPORATION) at a flow rate of 1.25 mL to equilibrate the column. 270 mL of the obtained rAAV9 virion-containing affinity fraction obtained in Example 7 was loaded onto the column at a flow rate of 1.25 mL/min. Next, a 20 mM Bis-Tris Propane buffer solution (pH 9.5) containing 4 mM MgCl₂ and 0.001% Pluronic^{™} F-68 having a volume 5 times larger than the column volume was passed through the column to wash the column. Next, a 20 mM Bis-Tris Propane buffer solution (pH 9.5) containing 4 mM MgCl₂, 0.001% Pluronic^{™} F-68, and 35 mM tetramethylammonium chloride having a volume 10 times larger than the column volume was passed through the column at a flow rate of 1.25 mL/min to elute the rAAV9 virions from the column. At this time, an absorptiometer was installed in a flow path downstream of a discharge port of the column, and absorbances at 260 nm and 280 nm of the eluate were continuously monitored to obtain a chromatogram. The eluate was recovered for each fraction corresponding to a peak on the chromatogram.

### [Example 11] Purification Process by Anion Exchange Column Chromatography (Condition D)

A 20 mM Bis-Tris Propane buffer solution (pH 9.5) containing 4 mM MgCl₂ and 0.001% Pluronic^{™} F-68 having a volume 8 times larger than a column volume was passed through a column (diameter of 0.8 cm and bed height of 20 cm) filled with 10 mL of TSKgel SuperQ-5PW(20) resin (TOSOH CORPORATION) at a flow rate of 1.25 mL to equilibrate the column. 270 mL of the obtained rAAV9 virion-containing affinity fraction obtained in Example 7 was loaded onto the column at a flow rate of 1.25 mL/min. Next, a 20 mM Bis-Tris Propane buffer solution (pH 9.5) containing 4 mM MgCl₂ and 0.001% Pluronic^{™} F-68 having a volume 5 times larger than the column volume was passed through the column to wash the column. Next, a 20 mM Bis-Tris Propane buffer solution (pH 9.5) containing 4 mM MgCl₂, 0.001% Pluronic^{™} F-68, and 40 mM tetramethylammonium chloride having a volume 10 times larger than the column volume was passed through the column at a flow rate of 1.25 mL/min to elute the rAAV9 virions from the column. At this time, an absorptiometer was installed in a flow path downstream of a discharge port of the column, and absorbances at 260 nm and 280 nm of the eluate were continuously monitored to obtain a chromatogram. The eluate was recovered for each fraction corresponding to a peak on the chromatogram.

### [Example 12] Purification Process by Anion Exchange Column Chromatography (Condition E)

A 20 mM Bis-Tris Propane buffer solution (pH 9.5) containing 4 mM MgCl₂ and 0.001% Pluronic^{™} F-68 having a volume 8 times larger than a column volume was passed through a column (diameter of 0.8 cm and bed height of 20 cm) filled with 10 mL of TSKgel SuperQ-5PW(20) resin (TOSOH CORPORATION) at a flow rate of 1.25 mL to equilibrate the column. 270 mL of the obtained rAAV9 virion-containing affinity fraction obtained in Example 7 was loaded onto the column at a flow rate of 1.25 mL/min. Next, a 20 mM Bis-Tris Propane buffer solution (pH 9.5) containing 4 mM MgCl₂ and 0.001% Pluronic^{™} F-68 having a volume 5 times larger than the column volume was passed through the column to wash the column. Next, a 20 mM Bis-Tris Propane buffer solution (pH 9.5) containing 4 mM MgCl₂, 0.001% Pluronic^{™} F-68, and 44 mM tetramethylammonium chloride having a volume 10 times larger than the column volume was passed through the column at a flow rate of 1.25 mL/min to elute the rAAV9 virions from the column. At this time, an absorptiometer was installed in a flow path downstream of a discharge port of the column, and absorbances at 260 nm and 280 nm of the eluate were continuously monitored to obtain a chromatogram. The eluate was recovered for each fraction corresponding to a peak on the chromatogram.

### [Example 13] Purification Process by Anion Exchange Column Chromatography (Condition F)

A 20 mM Bis-Tris Propane buffer solution (pH 9.5) containing 4 mM MgCl₂ and 0.001% Pluronic^{™} F-68 having a volume 8 times larger than a column volume was passed through a column (diameter of 0.8 cm and bed height of 20 cm) filled with 10 mL of TSKgel SuperQ-5PW(20) resin (TOSOH CORPORATION) at a flow rate of 1.25 mL to equilibrate the column. 270 mL of the obtained rAAV9 virion-containing affinity fraction obtained in Example 7 was loaded onto the column at a flow rate of 1.25 mL/min. Next, a 20 mM Bis-Tris Propane buffer solution (pH 9.5) containing 4 mM MgCl₂ and 0.001% Pluronic^{™} F-68 having a volume 5 times larger than the column volume was passed through the column to wash the column. Next, a 20 mM Bis-Tris Propane buffer solution (pH 9.5) containing 4 mM MgCl₂, 0.001% Pluronic^{™} F-68, and 48 mM tetramethylammonium chloride having a volume 10 times larger than the column volume was passed through the column at a flow rate of 1.25 mL/min to elute the rAAV9 virions from the column. At this time, an absorptiometer was installed in a flow path downstream of a discharge port of the column, and absorbances at 260 nm and 280 nm of the eluate were continuously monitored to obtain a chromatogram. The eluate was recovered for each fraction corresponding to a peak on the chromatogram.

### [Example 14] Quantification of rAAV9 Virions

The amount of rAAV9 genomes contained in each eluted fraction of anion exchange column chromatography under the condition A obtained in Example 8 was measured by the following droplet digital PCR method (ddPCR).

Pure water containing 1.6 µL of DNaseI (Takara Bio Inc.), 2 µL of 10 × DNaseI Buffer (Takara Bio Inc.), and 14.4 µL of 0.05% F-68 was added to 2 µL of the eluted fraction, incubation was performed at 37°C for 30 minutes to digest DNA not encapsulated in the rAAV9 virion, and then the mixture was appropriately diluted using TE buffer containing 0.05% F-68 to be used as a sample for droplet digital PCR.

FAM-labeled 20 x primer/probe mix containing a 1.0 µM forward primer (Primer SI-1, SEQ ID NO: 5), a 1.0 µM reverse primer (Primer SI-2, SEQ ID NO: 6), and a 0.25 µM probe (Probe SI-1, modified with FAM as a reporter dye at the 5' terminal and BHQ1 as a quencher dye at the 3' terminal of SEQ ID NO: 7, respectively) was prepared. In addition, HEX-labeled 20 x primer/probe mix containing a 1.0 µM forward primer (Primer SI-3, SEQ ID NO: 8), a 1.0 µM reverse primer (Primer SI-4, SEQ ID NO: 9), and a 0.25 µM probe (Probe SI-2, modified with HEX as a reporter dye at the 5' terminal and BHQ1 as a quencher dye at the 3' terminal of SEQ ID NO: 10, respectively) was prepared.

Pure water containing 10 µL of ddPCR Supermix for probes (no dUTP) (Bio-Rad Laboratories, Inc.), 1 µL of FAM labeled 20 × primer/probe mix, 1 µL of HEX labeled 20 × primer/probe mix, 2 µL of 5 M betaine solution (Sigma-Aldrich Inc.), and 4 µL of 0.05% F-68 was added to 2 µL of the sample solution for droplet digital PCR to prepare a PCR reaction solution. 20 µL of the PCR reaction solution and 70 µL of suspension droplets of Droplet Generator Oil for Probes (Bio-Rad Laboratories, Inc.) were prepared using Droplet generator (Bio-Rad Laboratories, Inc.). The droplets were subjected to QX200 Droplet Digital PCR system (Bio-Rad Laboratories, Inc.). The PCR conditions were a denaturation reaction (95°C, 10 minutes), 40 cycles of 3-step PCR (95°C, 30 seconds → 60°C, 60 seconds → 72°C, 15 seconds), and an inactivation treatment of a PCR enzyme (98°C, 10 minutes). FAM/HEX double-positive droplets were defined as rAAV positive droplets, and the amount of rAAV genomes (vg: viral genomes) was determined using QuantaSoft^{™} Version 1.7 (Bio-Rad Laboratories, Inc.). Note that the DNA region amplified in this PCR is an internal region of bovine growth hormone polyA and ITR. The amount (number) of rAAV9 genomes determined here theoretically matches the amount (number) of rAAV9 virions.

### [Example 15] Measurement of Proportion of rAAV9 Virions

A proportion of rAAV9 virions in the rAAV9 virions and empty capsids contained in each eluted fraction of each anion exchange resin purification obtained in each of Examples 8 to 13 was measured by the following method using an ultra-centrifuge for analysis (Optima-AUC, Beckman Coulter, Inc.). First, each eluted fraction was concentrated with Vivaspin Turbo15 (Sartorius AG) using an ultrafiltration membrane until the liquid volume reached 1 mL. Furthermore, the solution component of each eluted fraction was subjected to buffer exchange with a control buffer solution (8.1 mM disodium hydrogen phosphate, 1.5 mM potassium dihydrogen phosphate, 437 mM sodium chloride, 2.7 mM potassium chloride, and 0.001% Pluronic^{™} F-68), and the solution component subjected to the buffer exchange was used as a measurement sample. 400 µL of the measurement sample and 410 µL of a control buffer solution were injected into a centrifugal container, centrifugation was performed at 20,000 rpm (32,205 × g), and measurement of absorbances at wavelengths of 260 nm and 280 nm was performed 50 times every 30 seconds. The measurement result was read by analysis program sedfit for ultracentrifugation analysis and was analyzed by a continuous-size distribution model. Analysis parameters were set to resolution = 200, S min = 1, S max = 200, frictional ration = 1.2, partial specific volume = 0.73, buffer solution density = 1.0185, and buffer solution viscosity = 0.01047, and were optimized to the Lamm equation to analyze a distribution per sedimentation coefficient (S). An area of a peak corresponding to the rAAV9 virions and an area of a peak corresponding to the empty capsids were determined, and a proportion of the rAAV9 virions and a proportion of the empty capsids contained in each eluted fraction were determined. Here, the proportion of the rAAV9 virions is defined as a proportion (%) of rAAV9 virions when the total of rAAV9 virions and empty capsids is 100%, and a proportion of empty capsids is defined as a proportion (%) of empty capsids when the total of rAAV9 virions and empty capsids is 100%.

### [Example 16] Result: Proportions of Empty Capsids and rAAV Virions in Eluted Fractions (Condition A)

The chromatogram under the condition A obtained in Example 8 is illustrated in FIG. 5. The eluate was separated into the eluted fraction A1 to the eluted fraction A6 in the order of elution. The viral genome amount of rAAV9 virions contained in six fractions was measured by ddPCR. As a result, it was found that the amount of viral genomes contained in each fraction was 8.5 × 10¹² vg, 1.8 × 10¹³ vg, 1.6 × 10¹³ vg, 5.3 × 10¹² vg, 1.1 × 10¹² vg, and 2.2 × 10¹² vg in the order of the eluted fraction A1 to the eluted fraction A6, respectively, and rAAV9 virions were eluted in the eluted fraction A1 to the eluted fraction A3, particularly in the eluted fraction A2 and the eluted fraction A3.

Next, the proportions of the empty capsids and the rAAV9 virions contained in the eluted fraction A1 to the eluted fraction A3 were measured by the method shown in Example 15. The results are shown in Table 1. The proportions of the rAAV9 virions in the eluted fraction A1, the eluted fraction A2, and the eluted fraction A3 were 22.3%, 52.9%, and 56.0%, respectively. This result indicates that rAAV9 virions having a proportion of rAAV9 virions of 20% or more can be obtained by recovering the eluted fractions A1 to A3, and rAAV9 virions having a proportion of rAAV9 virions of approximately 50% or more can be obtained by recovering the eluted fractions A2 and A3. In addition, the amount of rAAV9 virions when the eluted fractions A1 to A3 are recovered is 4.3 × 10¹³, and the amount of rAAV9 virions when the eluted fractions A2 and A3 are recovered is 3.4 × 10¹³. Here, a concentration of tetramethylammonium chloride contained in the eluate when the eluted fractions A1 to A3 were eluted was approximately 30 mM to 50 mM.

**[Table 1]**

| Table 1 Proportions of rAAV9 virions and empty capsids contained in eluted fractions A1 to A3 | | |
|---|---|---|
| Eluted fraction | Proportion of empty capsids (%) | Proportion of rAAV9 virions (%) |
| A1 | 77.7 | 22.3 |
| A2 | 47.1 | 52.9 |
| A3 | 44.0 | 56.0 |

### [Example 17] Result: Proportions of Empty Capsids and rAAV Virions in Eluted Fractions (Condition B)

The chromatogram under the condition B obtained in Example 9 is illustrated in FIG. 6. On the chromatogram, there are two peaks (B1 and B2) appearing in the eluate when passing through a buffer solution containing a 30 mM tetramethylammonium salt and a peak (B3) appearing in the eluate when passing through a buffer solution containing a 48 mM tetramethylammonium salt. The eluates corresponding to B1, B2, and B3 were fractionated to obtain an eluted fraction B1, an eluted fraction B2, and an eluted fraction B3, respectively.

Proportions of empty capsids and rAAV9 virions contained in the eluted fraction B1 to the eluted fraction B3 were measured by the method shown in Example 15. The proportions of the rAAV9 virions in the eluted fractions B1, B2, and B3 were 1.1%, 58.0%, and 76.4%, respectively (Table 2). This result indicates that rAAV9 virions having a proportion of rAAV9 virions of 50% or more or 55% or more (for example, 58%) can be obtained by recovering the eluted fractions B2 and B3, and rAAV9 virions having a proportion of rAAV9 virions of 70% or more or 75% or more (for example, 76%) can be obtained by recovering only the eluted fraction B3.

**[Table 2]**

| Table 2 Proportions of rAAV9 virions and empty capsids contained in eluted fractions B1 to B3 | | |
|---|---|---|
| Eluted fraction | Proportion of empty capsids (%) | Proportion of rAAV9 virions (%) |
| B1 | 98.9 | 1.1 |
| B2 | 42.0 | 58.0 |
| B3 | 23.6 | 76.4 |

That is, in the anion exchange column chromatography, the column is washed by passing a buffer solution containing a 30 mM tetramethylammonium salt through the column on which the rAAV9 virions are adsorbed, and then only the eluate (corresponding to the eluted fraction B3) obtained by passing a buffer solution containing a 48 mM tetramethylammonium salt is recovered, such that an rAAV9 virion solution having a proportion of rAAV9 virions of 70% or more or 75% or more (for example, 75% or 76%) can be obtained.

Both of the eluted fractions B1 and B2 are eluates obtained by passing a buffer solution containing a 30 mM tetramethylammonium salt through a column adsorbing rAAV9 virions. Peaks B1 and B2 are clearly separated on the chromatogram (FIG. 6). Therefore, the eluted fractions B1 and B2 can be separated and recovered by continuously monitoring the absorbance of the eluate obtained by passing a buffer solution containing a 30 mM tetramethylammonium salt. An rAAV9 virion solution having a proportion of rAAV9 virions of 50% or more or 55% or more (for example, 58%) can be obtained by discarding the eluted fraction B1 and recovering the eluted fractions B2 and B3. Furthermore, an rAAV9 virion solution having a proportion of rAAV9 virions of 70% or more or 75% or more (for example, 76%) can be obtained by only recovering the eluted fraction B3.

### [Example 18] Result: Proportions of Empty Capsids and rAAV Virions in Eluted Fractions (Condition C)

The chromatogram under the condition C obtained in Example 10 is illustrated in FIG. 7. On the chromatogram, there are two peaks (C1 and C2) appearing in the eluate when passing through a buffer solution containing a 35 mM tetramethylammonium salt. The eluates corresponding to C1 and C2 were fractionated to obtain an eluted fraction C1 and an eluted fraction C2, respectively.

Proportions of empty capsids and rAAV9 virions contained in the eluted fractions C1 and C2 were measured by the method shown in Example 15. The proportions of the rAAV9 virions in the eluted fractions C1 and C2 were 12.1% and 80.0%, respectively (Table 3). The result indicates that rAAV9 virions having a proportion of rAAV9 virions of 75% or more (for example, 78% or 80%) can be obtained by recovering only the eluted fraction C3.

**[Table 3]**

| Table 3 Proportions of rAAV9 virions and empty capsids contained in eluted fractions C1 and C2 | | |
|---|---|---|
| Eluted fraction | Proportion of empty capsids (%) | Proportion of rAAV9 virions (%) |
| C1 | 87.9 | 12.1 |
| C2 | 20.0 | 80.0 |

Peaks C1 and C2 are clearly separated on the chromatogram (FIG. 7). Therefore, the eluted fractions C1 and C2 can be separated and recovered by continuously monitoring the absorbance of the eluate obtained by passing a buffer solution containing a 35 mM tetramethylammonium salt. An rAAV9 virion solution having a proportion of rAAV9 virions of 75% or more (for example, 78% or 80%) can be obtained by discarding the eluted fraction C1 and recovering only the eluted fraction C2.

### [Example 19] Result: Proportions of Empty Capsids and rAAV Virions in Eluted Fractions (Condition D)

The chromatogram under the condition D obtained in Example 11 is illustrated in FIG. 8. On the chromatogram, there are two peaks (D1 and D2) appearing in the eluate when passing through a buffer solution containing a 40 mM tetramethylammonium salt. The eluates corresponding to D1 and D2 were fractionated to obtain an eluted fraction D1 and an eluted fraction D2, respectively.

Proportions of empty capsids and rAAV9 virions contained in the eluted fractions D1 and D2 were measured by the method shown in Example 15. The proportions of the rAAV9 virions in the eluted fractions D1 and D2 were 16.2% and 83.5%, respectively (Table 4). The result indicates that rAAV9 virions having a proportion of rAAV9 virions of 75% or more or 80% or more (for example, 79% or 83%) can be obtained by recovering only the eluted fraction D2.

**[Table 4]**

| Table 4 Proportions of rAAV9 virions and empty capsids contained in eluted fractions D1 and D2 | | |
|---|---|---|
| Eluted fraction | Proportion of empty capsids (%) | Proportion of rAAV9 virions (%) |
| D1 | 83.8 | 16.2 |
| D2 | 16.5 | 83.5 |

Peaks D1 and D2 are clearly separated on the chromatogram (FIG. 8). Therefore, the eluted fractions D1 and D2 can be separated and recovered by continuously monitoring the absorbance of the eluate obtained by passing a buffer solution containing a 40 mM tetramethylammonium salt. An rAAV9 virion solution having a proportion of rAAV9 virions of 75% or more or 80% or more (for example, 79% or 83%) can be obtained by discarding the eluted fraction D1 and recovering only the eluted fraction D2.

### [Example 20] Result: Proportions of Empty Capsids and rAAV Virions in Eluted Fractions (Condition E)

The chromatogram under the condition E obtained in Example 12 is illustrated in FIG. 9. On the chromatogram, there are two peaks (E1 and E2) appearing in the eluate when passing through a buffer solution containing a 44 mM tetramethylammonium salt. The eluates corresponding to E1 and E2 were fractionated to obtain an eluted fraction E1 and an eluted fraction E2, respectively.

Proportions of empty capsids and rAAV9 virions contained in the eluted fractions E1 and E2 were measured by the method shown in Example 15. The proportions of the rAAV9 virions in the eluted fractions E1 and E2 were 30.8% and 85.4%, respectively (Table 5). The result indicates that rAAV9 virions having a proportion of rAAV9 virions of 80% or more (for example, 83% or 85%) can be obtained by recovering only the eluted fraction E2.

**[Table 5]**

| Table 5 Proportions of rAAV9 virions and empty capsids contained in eluted fractions E1 and E2 | | |
|---|---|---|
| Eluted fraction | Proportion of empty capsids (%) | Proportion of rAAV9 virions (%) |
| E1 | 69.2 | 30.8 |
| E2 | 14.6 | 85.4 |

Peaks E1 and E2 are clearly separated on the chromatogram (FIG. 9). Therefore, the eluted fractions E1 and E2 can be separated and recovered by continuously monitoring the absorbance of the eluate obtained by passing a buffer solution containing a 44 mM tetramethylammonium salt. An rAAV9 virion solution having a proportion of rAAV9 virions of 80% or more (for example, 83% or 85%) can be obtained by discarding the eluted fraction E1 and recovering only the eluted fraction E2.

### [Example 21] Result: Proportions of Empty Capsids and rAAV Virions in Eluted Fractions (Condition F)

The chromatogram under the condition F obtained in Example 13 is illustrated in FIG. 10. On the chromatogram, there are two peaks (F1 and F2) appearing in the eluate when passing through a buffer solution containing a 48 mM tetramethylammonium salt. The eluates corresponding to F1 and F2 were fractionated to obtain an eluted fraction F1 and an eluted fraction F2, respectively.

Proportions of empty capsids and rAAV9 virions contained in the eluted fractions F1 and F2 were measured by the method shown in Example 15. The proportions of the rAAV9 virions in the eluted fractions F1 and F2 were 28.7% and 87.0%, respectively (Table 6). The result indicates that rAAV9 virions having a proportion of rAAV9 virions of 80% or more or 85% or more (for example, 84% or 87%) can be obtained by recovering only the eluted fraction F2.

**[Table 6]**

| Table 6 Proportions of rAAV9 virions and empty capsids contained in eluted fractions F1 and F2 | | |
|---|---|---|
| Eluted fraction | Proportion of empty capsids (%) | Proportion of rAAV9 virions (%) |
| F1 | 71.3 | 28.7 |
| F2 | 13.0 | 87.0 |

Peaks F1 and F2 are clearly separated on the chromatogram (FIG. 10). Therefore, the eluted fractions F1 and F2 can be separated and recovered by continuously monitoring the absorbance of the eluate obtained by passing a buffer solution containing a 48 mM tetramethylammonium salt. An rAAV9 virion solution having a proportion of rAAV9 virions of 80% or more or 85% or more (for example, 84% or 87%) can be obtained by discarding the eluted fraction F1 and recovering only the eluted fraction F2.

### Industrial Applicability

According to one embodiment of the present invention, it is possible to efficiently produce rAAV9 virions in which DNA containing a nucleotide sequence encoding a foreign protein is packaged, such that rAAV9 virions can be stably supplied to a medical institution and the like.

### Sequence Listing Free Text

SEQ ID NO: 1: Nucleotide sequence and synthetic sequence containing SalI site, RsrII site, ColE1 ori, ampicillin resistance gene, BsiWI site, BstZ17I site, and BsrGI site
SEQ ID NO: 2: Nucleotide sequence and synthetic sequence containing ClaI site, human CMV enhancer, chicken β-actin promoter, chicken β-actin/MVM chimeric intron, palmitoyl-protein thioesterase-1 (PPT1) gene, woodchuck hepatitis virus posttranscriptional regulatory element (WPRE), bovine growth hormone polyA sequence, and BglII site
SEQ ID NO: 3: Nucleotide sequence and synthetic sequence containing AfeI site, RsrII site, BsrGI site, AvrII site, ampicillin resistance gene, ColE1 ori, RsrII site, 5' portion of Rep region of serotype 2 (containing p5 promoter), and SacII site
SEQ ID NO: 4: Nucleotide sequence and synthetic sequence containing HindIII site, 3' portion of Rep region of AAV of serotype 2, Cap region of serotype 9, p5 promoter, RsrII site, and BsrGI site
SEQ ID NO: 5: Nucleotide sequence and synthetic sequence of forward primer and primer SI-1
SEQ ID NO: 6: Nucleotide sequence and synthetic sequence of reverse primer and primer SI-2
SEQ ID NO: 7: Nucleotide sequence and synthetic sequence of probe SI-1
SEQ ID NO: 8: Nucleotide sequence and synthetic sequence of forward primer and primer SI-3
SEQ ID NO: 9: Nucleotide sequence and synthetic sequence of reverse primer and primer SI-4
SEQ ID NO: 10: Nucleotide sequence and synthetic sequence of probe SI-2

## Claims

1. A production method of rAAV9 virions, the production method comprising:
(a) culturing mammalian cells containing an introduced AAV vector in a medium to release rAAV9 virions into the medium;
(b) recovering a culture supernatant from the medium; and
(c) purifying the rAAV9 virions from the recovered culture supernatant by affinity column chromatography using a material having an affinity for an AAV capsid protein as a stationary phase and anion column chromatography using an anion exchanger as a stationary phase.

2. The production method according to claim 1, wherein the stationary phase used for the affinity column chromatography has an affinity for an AAV capsid protein of serotype 9.

3. The production method according to claim 1 or 2, wherein the anion exchanger is a strong anion exchanger.

4. The production method according to claim 3, wherein the strong anion exchanger contains a quaternary amine.

5. The production method according to any one of claims 1 to 4, wherein in the anion exchange column chromatography, the rAAV9 virions are adsorbed on the column, and then the rAAV9 virions are eluted from the column by a buffer solution containing a quaternary ammonium salt to recover a fraction containing the rAAV9 virions.

6. The production method according to claim 5, wherein a pH of the buffer solution is 8.5 to 10.5.

7. The production method according to claim 5 or 6, wherein a buffer contained in the buffer solution is selected from the group consisting of tris-hydrochloric acid, Bis-Tris, Bis-Tris propane, bicine (N,N-bis(2-hydroxyethyl)glycine), 4-2-hydroxyethyl-1-piperazineethanesulfonic acid (HEPES), 3-{[tris(hydroxymethyl)methyl]amino}propane ethanesulfonic acid (TAPS), tricine (N-tris(hydroxymethyl)methylglycine), and a combination of any two or more thereof.

8. The production method according to claim 5 or 6, wherein a buffer contained in the buffer solution is Bis-Tris propane.

9. The production method according to claim 7 or 8, wherein a concentration of the buffer is 10 mM to 30 mM.

10. The production method according to any one of claims 5 to 9, wherein the buffer solution contains a neutral salt.

11. The method according to claim 10, wherein the neutral salt is magnesium chloride.

12. The method according to claim 10 or 11, wherein a concentration of the neutral salt is 1 mM to 10 mM.

13. The production method according to any one of claims 5 to 12, wherein the buffer solution contains a nonionic surfactant.

14. The production method according to claim 13, wherein the nonionic surfactant is selected from the group consisting of a polyoxyethylene-polyoxypropylene block copolymer, a polysorbate, a poloxamer, and a combination of any two or more thereof.

15. The production method according to claim 13, wherein the nonionic surfactant is selected from the group consisting of polysorbate 20, polysorbate 80, poloxamer 188, and a combination of any two or more thereof.

16. The production method according to any one of claims 13 to 15, wherein a concentration of the nonionic surfactant is 0.0002 to 0.01% (w/v).

17. The production method according to any one of claims 5 to 16, wherein the quaternary ammonium salt contained in the buffer solution is a tetramethylammonium salt.

18. The production method according to claim 17, wherein the tetramethylammonium salt is tetramethylammonium chloride.

19. The production method according to any one of claims 5 to 18, wherein the adsorbed rAAV9 virions are eluted by the buffer solution containing the quaternary ammonium salt at a concentration of 20 mM to 60 mM.

20. The production method according to any one of claims 5 to 18, wherein the adsorbed rAAV9 virions are eluted by the buffer solution containing the quaternary ammonium salt at a concentration of 35 mM to 55 mM.

21. The production method according to any one of claims 5 to 20, wherein in the anion exchange column chromatography, a fraction containing the rAAV9 virions is recovered so that a ratio of the rAAV9 virions and empty capsids is 2 : 8 to 10 : 0.

22. The production method according to any one of claims 5 to 20, wherein in the anion exchange column chromatography, a fraction containing the rAAV9 virions is recovered so that a ratio of the rAAV9 virions and empty capsids is 5 : 5 to 10 : 0.

23. The production method according to any one of claims 5 to 20, wherein in the anion exchange column chromatography, a fraction containing the rAAV9 virions is recovered so that a ratio of the rAAV9 virions and empty capsids is 7 : 3 to 10 : 0.

24. A production method of rAAV9 virions, the production method comprising loading an aqueous solution containing rAAV9 virions and empty capsids onto anion exchange column chromatography using an anion exchanger as a stationary phase to adsorb the rAAV9 virions on the anion exchanger, and then eluting the rAAV9 virions from the anion exchanger by a buffer solution containing a quaternary ammonium salt to recover a fraction containing the rAAV9 virions.

25. The production method according to claim 24, wherein a pH of the buffer solution is 8.5 to 10.5.

26. The production method according to claim 24 or 25, wherein a buffer contained in the buffer solution is selected from the group consisting of tris-hydrochloric acid, Bis-Tris, Bis-Tris propane, bicine (N,N-bis(2-hydroxyethyl)glycine), 4-2-hydroxyethyl-1-piperazineethanesulfonic acid (HEPES), 3-{[tris(hydroxymethyl)methyl]amino}propane ethanesulfonic acid (TAPS), tricine (N-tris(hydroxymethyl)methylglycine), and a combination of any two or more thereof.

27. The production method according to claim 24 or 25, wherein a buffer contained in the buffer solution is Bis-Tris Propane.

28. The production method according to claim 26 or 27, wherein a concentration of the buffer is 10 mM to 30 mM.

29. The production method according to any one of claims 24 to 28, wherein the buffer solution contains a neutral salt.

30. The method according to claim 29, wherein the neutral salt is magnesium chloride.

31. The method according to claim 29 or 30, wherein a concentration of the neutral salt is 1 mM to 10 mM.

32. The production method according to any one of claims 24 to 31, wherein the buffer solution contains a nonionic surfactant.

33. The production method according to claim 32, wherein the nonionic surfactant is selected from the group consisting of a polyoxyethylene-polyoxypropylene block copolymer, a polysorbate, a poloxamer, and a combination of any two or more thereof.

34. The production method according to claim 32, wherein the nonionic surfactant is selected from the group consisting of polysorbate 20, polysorbate 80, poloxamer 188, and a combination of any two or more thereof.

35. The production method according to any one of claims 32 to 34, wherein a concentration of the nonionic surfactant is 0.0002 to 0.01% (w/v).

36. The production method according to claims 24 to 35, wherein the quaternary ammonium salt contained in the buffer solution is a tetramethylammonium salt.

37. The method according to claim 36, wherein the tetramethylammonium salt is tetramethylammonium chloride.

38. The production method according to any one of claims 24 to 37, wherein the adsorbed rAAV9 virions are eluted by the buffer solution containing the quaternary ammonium salt at a concentration of 20 mM to 60 mM.

39. The production method according to any one of claims 24 to 37, wherein the adsorbed rAAV9 virions are eluted by the buffer solution containing the quaternary ammonium salt at a concentration of 35 mM to 55 mM.

40. The production method according to any one of claims 24 to 39, wherein a fraction containing the rAAV9 virions is recovered so that a ratio of the rAAV9 virions and empty capsids is 2 : 8 to 10 : 0.

41. The production method according to any one of claims 24 to 39, wherein a fraction containing the rAAV9 virions is recovered so that a ratio of the rAAV9 virions and empty capsids is 5 : 5 to 10 : 0.

42. The production method according to any one of claims 24 to 39, wherein a fraction containing the rAAV9 virions is recovered so that a ratio of the rAAV9 virions and empty capsids is 7 : 3 to 10 : 0.
